(19) Europäisches Patentamt / European Patent Office / Office européen des brevets

(11) EP 4 389 160 A1

(12) **EUROPEAN PATENT APPLICATION**
published in accordance with Art. 153(4) EPC

(43) Date of publication:
**26.06.2024 Bulletin 2024/26**

(21) Application number: **22858603.8**

(22) Date of filing: **29.06.2022**

(51) International Patent Classification (IPC):
***A61L 9/01*** *(2006.01)* ***A61L 9/012*** *(2006.01)*
***C08J 3/24*** *(2006.01)*

(52) Cooperative Patent Classification (CPC):
**A61Q 15/00; A61K 8/33; A61K 8/347;**
**A61Q 17/005;** A61K 2800/57

(86) International application number:
**PCT/KR2022/009359**

(87) International publication number:
**WO 2023/022363 (23.02.2023 Gazette 2023/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.08.2021 KR 20210110455**

(71) Applicant: **LG CHEM, LTD.**
**Yeongdeungpo-gu,**
**Seoul 07336 (KR)**

(72) Inventors:
- **HUR, Yoon Hyung**
  **Daejeon 34122 (KR)**
- **KANG, Soonhee**
  **Daejeon 34122 (KR)**
- **BAEK, Leehyeon**
  **Daejeon 34122 (KR)**
- **SEO, Ue Ryung**
  **Daejeon 34122 (KR)**
- **LEE, Ji Seok**
  **Daejeon 34122 (KR)**
- **JUNG, Seonjung**
  **Daejeon 34122 (KR)**
- **CHOI, Hyungsam**
  **Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

(54) **ANTIBACTERIAL AND DEODORIZING COMPOSITION, AND PREPARATION METHOD THEREFOR**

(57) The present specification relates to an antibacterial deodorant composition comprising: a first compound of Chemical Formula 1; and a second compound different from the first compound, in which the first compound is cross-linked to at least a portion of the second compound, a content of guaiacol is 300 ng or less, a content of 3-methylbutanal is 250 ng or less, and a content of diacetyl is 30 ng or less when the antibacterial deodorant composition is evaluated for deodorization by Method A, and a preparation method thereof.

[Figure 1]

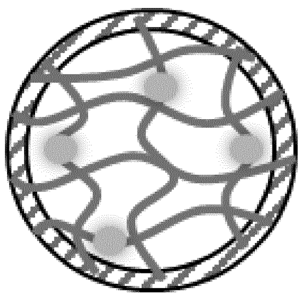

## Description

[Technical Field]

This application claims priority to and the benefit of Korean Patent Application No. 10-2021-0110455 filed in the Korean Intellectual Property Office on August 20, 2021, the entire contents of which are incorporated herein by reference.

The present specification relates to an antibacterial deodorant composition and a preparation method thereof.

[Background Art]

Recently, various products such as daily supplies or hygiene products are required to have antibacterial and deodorant properties. These products need to be able to have excellent antibacterial and deodorant properties while maintaining the properties suitable for their respective functions. For example, hygiene products such as diapers and sanitary napkins are products for which absorbency is most important, and since these products come in direct contact with the human body, the importance of antibacterial and deodorant effects is increasing.

However, when the products are prepared by adding an antibacterial agent or deodorant agent in order to impart antibacterial and deodorant effects, there is a problem in that absorption capacity deteriorates or antibacterial or deodorizing power is not maintained.

Therefore, there is a need for developing a composition which provides antibacterial and deodorant effects while having sufficient absorption capacity and retention capacity.

[Detailed Description of the Invention]

[Technical Problem]

The present specification has been made in an effort to provide an antibacterial deodorant composition and a preparation method thereof.

[Technical Solution]

An exemplary embodiment of the present specification provides an antibacterial deodorant composition comprising: a first compound of the following Chemical Formula 1; and a second compound different from the first compound, in which the first compound is cross-linked to at least a portion of the second compound, a content of guaiacol is 300 ng or less, a content of 3-methylbutanal is 250 ng or less, and a content of diacetyl is 30 ng or less when the antibacterial deodorant composition is evaluated for deodorization, and the deodorization evaluation is determined by the following Method A.

[Chemical Formula 1]

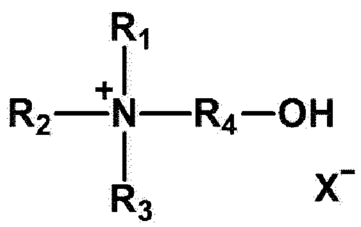

In Chemical Formula 1,

$R_1$ to $R_3$ are each independently an alkyl group having 1 to 12 carbon atoms, which is unsubstituted or substituted with a hydroxyl group,
at least one of $R_1$ to $R_3$ is an alkyl group having 8 to 12 carbon atoms,
$R_4$ is an alkylene group having 1 to 6 carbon atoms,
X is a halogen,
[Method A]

After 1 g of the antibacterial deodorant composition is put into a 500 ml Lab bottle, 25 ml of artificial urine inoculated with microorganisms was injected into the composition, the resulting mixture was cultured at 35°C for 24 hours, and then guaiacol, 3-methylbutanal, and diacetyl components are each captured in an adsorption tube, and the mass of each captured component is analyzed using GC/MS.

Another exemplary embodiment of the present specification provides a method for preparing an antibacterial deodorant composition, the method comprising: (a) preparing a mixture of the first compound of Chemical Formula 1 and a second compound different from the first compound; and (b) cross-linking the mixture.

Still another exemplary embodiment of the present specification provides a deodorant composition comprising the first compound of Chemical Formula 1.

[Advantageous Effects]

The antibacterial deodorant composition according to the present specification comprises a form in which the surface is cross-linked by an alcohol-based antibacterial deodorant monomer comprising quaternary ammonium to provide not only excellent antibacterial and deodorant effects, but also a composition with high centrifuge retention capacity and absorbing under pressure.

Since the antibacterial deodorant monomer of the present invention is comprised in the composition in a cross-linked form rather than in the form of an additive, the antibacterial deodorant composition has excellent durability and does not cause any elution problem.

In particular, the antibacterial deodorant monomer of the present invention has both hydrophobicity and hydrophilicity by having a long alkyl group of 8 to 12 carbon atoms and a hydroxy group, so the monomer is suitable for surface treatment and antibacterial and deodorant effect can also be obtained.

Further, by cross-linking the antibacterial deodorant monomer at 150°C to 220°C for more than 20 minutes, there is an effect in which the desired antibacterial power and deodorizing power are obtained and the centrifuge retention capacity and absorbing under pressure are improved.

[Brief Description of Drawings]

FIG. 1 illustrates a particle structure of the antibacterial deodorant composition of the present invention.
FIGS. 2 to 8 are NMR data of the antibacterial deodorant monomer prepared in Preparation Example 1.

[Best Mode]

When one part "comprises" one constituent element in the present specification, unless otherwise specifically described, this does not mean that another constituent element is excluded, but means that another constituent element may be further comprised.

In the present specification, an alkyl group may be straight-chained or branched.

In the present specification, an alkylene group means a divalent alkyl group, and may be straight-chained or branched.

In the present specification, a halogen may be fluorine (F), chlorine (Cl), bromine (Br), or iodine (I).

Hereinafter, the present specification will be described in more detail.

An exemplary embodiment of the present specification provides an antibacterial deodorant composition comprising: a first compound of the following Chemical Formula 1; and a second compound different from the first compound, in which the first compound is cross-linked to at least a portion of the second compound, a content of guaiacol is 300 ng or less, a content of 3-methylbutanal is 250 ng or less, and a content of diacetyl is 30 ng or less when the antibacterial deodorant composition is evaluated for deodorization, and the deodorization evaluation is determined by the following Method A.

[Chemical Formula 1]

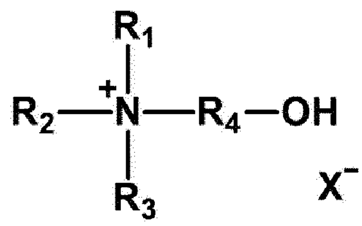

In Chemical Formula 1,

$R_1$ to $R_3$ are each independently an alkyl group having 1 to 12 carbon atoms, which is unsubstituted or substituted with a hydroxyl group,
at least one of $R_1$ to $R_3$ is an alkyl group having 8 to 12 carbon atoms,
$R_4$ is an alkylene group having 1 to 6 carbon atoms,
X is a halogen,
[Method A]

After 1 g of the antibacterial deodorant composition is put into a 500 ml Lab bottle, 25 ml of artificial urine inoculated with microorganisms was injected into the composition, the resulting mixture was cultured at 35°C for 24 hours, and then guaiacol, 3-methylbutanal, and diacetyl components are each captured in an adsorption tube, and the mass of each captured component is analyzed using GC/MS.

The antibacterial deodorant composition according to an exemplary embodiment of the present specification provides excellent centrifuge retention capacity and absorbing under pressure while having antibacterial and deodorant effects.

Specifically, the antibacterial deodorant composition of the present invention has a guaiacol content of 300 ng or less, a 3-methylbutanal content of 250 ng or less, and a diacetyl content of 30 ng or less when the antibacterial deodorant composition is evaluated for deodorization by comprising a form in which the first compound of Chemical Formula 1 is cross-linked to at least a portion of the second compound.

In an exemplary embodiment of the present specification, the deodorization evaluation is determined by the following Method A.

[Method A]

After 1 g of the antibacterial deodorant composition is put into a 500 ml Lab bottle, 25 ml of artificial urine inoculated with microorganisms was injected into the composition, the resulting mixture was cultured at 35°C for 24 hours, and then guaiacol, 3-methylbutanal, and diacetyl components are each captured in an adsorption tube, and the mass of each captured component is analyzed using GC/MS.

In an exemplary embodiment of the present specification, the deodorization evaluation may be performed on artificial urine odor components such as DMDS+DMTS and p-cresol in addition to guaiacol, diacetyl and 3-methylbutanal. That is, in Method A, the mass is analyzed by capturing DMDS+DMTS or p-cresol instead of guaiacol, diacetyl, or 3-methylbutanal.

In an exemplary embodiment of the present specification, when the antibacterial deodorant composition is evaluated for deodorization, the content of guaiacol may be 300 ng or less, 260 ng or less, or 251 ng or less, preferably 200 ng or less, 199 ng or less, or 180 ng or less, and more preferably 173 ng or less, 170 ng or less, or 168 ng or less. In addition, the lower limit thereof is not limited, but may be, for example, 0 ng or more. The smaller content means that the deodorizing power against guaiacol is higher.

In an exemplary embodiment of the present specification, when the antibacterial deodorant composition is evaluated for deodorization, the content of 3-methylbutanal may be 250 ng or less, 210 ng or less, or 205 ng or less, preferably 180 ng or less, or 150 ng or less, and more preferably 130 ng or less, 129 ng or less, 124 ng or less, 102 ng or less, or 99 ng or less. Furthermore, the lower limit thereof is not limited, but may be, for example, 0 ng or more. The smaller content means that the deodorizing power against 3-methylbutanal is higher.

In an exemplary embodiment of the present specification, when the antibacterial deodorant composition is evaluated for deodorization, the content of diacetyl may be 30 ng or less, 29 ng or less, or 28 ng or less, preferably 25 ng or less, or 24 ng or less, and more preferably 21 ng or less, or 20 ng or less. Further, the lower limit is not limited, but may be, for example, 0 ng or more. The smaller content means that the deodorizing power against diacetyl is higher.

In an exemplary embodiment of the present specification, the artificial urine used for the deodorization evaluation may be prepared with the same composition as that in the ESSITY document (J Wound Ostomy Continence Nurs.

2019;46(6):519-523.). Among the materials used for deodorization evaluation, those that can be sterilized may be sterilized using an autoclave, and those that cannot be sterilized at high temperatures may be sterilized using a 0.20 μm membrane filter.

In an exemplary embodiment of the present specification, examples of microorganisms used for the deodorization evaluation comprise Escberichia Coli (E. coli, CCUG 3274), Proteus mirabilis (P. mirabilis CCUG 4637), Enterobacter Cloacae (E. cloacae, CCUG 71839), and the like, but are not limited thereto.

In an exemplary embodiment of the present specification, the microorganisms used for the deodorizing evaluation may be mixed bacteria. That is, one or more types of microorganisms are mixed and may be used for deodorization evaluation.

In an exemplary embodiment of the present specification, the concentration of one or more microorganisms used for the deodorization evaluation may be each $10^5$ CFU/ml to $10^6$ CFU/ml.

In an exemplary embodiment of the present specification, a mixture of $10^5$ CFU/ml E. coli, $10^6$ CFU/ml Proteus mirabilis and $10^6$ CFU/ml E. cloacae may be used for the deodorization evaluation.

In an exemplary embodiment of the present specification, the first compound is a monomer having antibacterial and deodorant properties and serves as a surface cross-linking agent.

The first compound represented by Chemical Formula 1 is a quaternary ammonium-based compound with antibacterial properties, and the cations of the ammonium molecule are electrostatically adsorbed to the anion sites on the surface of a microorganism cell and kill the cells by physicochemically destructing the cell surface structure by hydrophobic interactions. The hydrophobicity of the first compound varies depending on the number of carbon atoms of the alkyl group linked to the quaternary ammonium, resulting in varying antibacterial properties.

In an exemplary embodiment of the present specification, $R_1$ to $R_3$ of Chemical Formula 1 are each independently an alkyl group having 1 to 12 carbon atoms, which is unsubstituted or substituted with a hydroxyl group, and at least one of $R_1$ to $R_3$ is an alkyl group having 8 to 12 carbon atoms.

In an exemplary embodiment of the present specification, $R_3$ may be an alkyl group having 8 to 12 carbon atoms.

In an exemplary embodiment of the present specification, $R_3$ may be a straight-chained alkyl group having 8 to 12 carbon atoms.

In an exemplary embodiment of the present specification, $R_3$ may be a straight-chained alkyl group having 10 to 12 carbon atoms.

In an exemplary embodiment of the present specification, $R_3$ may be a straight-chained alkyl group having 8 carbon atoms.

In an exemplary embodiment of the present specification, $R_3$ may be a straight-chained alkyl group having 10 carbon atoms.

In an exemplary embodiment of the present specification, $R_3$ may be a straight-chained alkyl group having 12 carbon atoms.

When the number of carbon atoms in $R_3$ is less than 8, for example, 4, the antibacterial and deodorant properties of the antibacterial and deodorant composition deteriorate.

In an exemplary embodiment of the present specification, $R_1$ and $R_2$ may be each independently an alkyl group having 1 to 12 carbon atoms, which is unsubstituted or substituted with a hydroxyl group.

In an exemplary embodiment of the present specification, $R_1$ and $R_2$ may be each independently an alkyl group having 1 to 8 carbon atoms, which is unsubstituted or substituted with a hydroxyl group.

In an exemplary embodiment of the present specification, $R_1$ and $R_2$ may be each independently an alkyl group having 1 to 5 carbon atoms, which is unsubstituted or substituted with a hydroxyl group.

In an exemplary embodiment of the present specification, $R_1$ and $R_2$ may be each independently a straight-chained alkyl group having 1 to 5 carbon atoms, which is unsubstituted or substituted with a hydroxyl group.

In an exemplary embodiment of the present specification, $R_1$ and $R_2$ may be each independently a straight-chained alkyl group having 1 to 3 carbon atoms, which is unsubstituted or substituted with a hydroxyl group.

In an exemplary embodiment of the present specification, at least one of $R_1$ and $R_2$ may be an alkyl group having 1 to 5 carbon atoms.

In an exemplary embodiment of the present specification, at least one of $R_1$ and $R_2$ may be a straight-chained alkyl group having 1 to 5 carbon atoms.

In an exemplary embodiment of the present specification, at least one of $R_1$ and $R_2$ may be a straight-chained alkyl group having 1 to 3 carbon atoms.

In an exemplary embodiment of the present specification, at least one of $R_1$ and $R_2$ may be a methyl group.

In an exemplary embodiment of the present specification, any one of $R_1$ and $R_2$ may be an alkyl group having 1 to 5 carbon atoms, which is unsubstituted or substituted with a hydroxyl group.

In an exemplary embodiment of the present specification, any one of $R_1$ and $R_2$ may be an alkyl group having 1 to 5 carbon atoms, which is substituted with a hydroxyl group.

In an exemplary embodiment of the present specification, any one of $R_1$ and $R_2$ may be an alkyl group having

1 to 5 carbon atoms.

In an exemplary embodiment of the present specification, $R_1$ may be an alkyl group having 1 to 12 carbon atoms.

In an exemplary embodiment of the present specification, $R_2$ may be an alkyl group having 1 to 12 carbon atoms, which is unsubstituted or substituted with a hydroxyl group.

In an exemplary embodiment of the present specification, $R_4$ of Chemical Formula 1 may be a straight-chained alkylene group having 1 to 6 carbon atoms.

In an exemplary embodiment of the present specification, $R_4$ may be a straight-chained alkylene group having 1 to 4 carbon atoms.

In an exemplary embodiment of the present specification, $R_4$ may be a straight-chained alkylene group having 2 to 4 carbon atoms.

In an exemplary embodiment of the present specification, $R_4$ may be a straight-chained alkylene group having 2 carbon atoms.

In an exemplary embodiment of the present specification, $R_4$ may be a straight-chained alkylene group having 3 carbon atoms.

In an exemplary embodiment of the present specification, $R_4$ may be a straight-chained alkylene group having 4 carbon atoms.

In an exemplary embodiment of the present specification, X of Chemical Formula 1 may be Br or Cl.

In an exemplary embodiment of the present specification, the first compound may be any one of the following compounds.

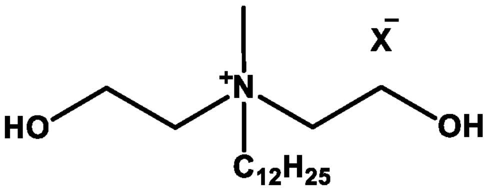
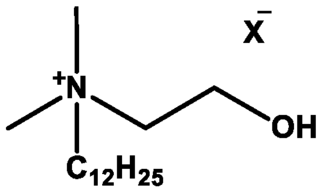
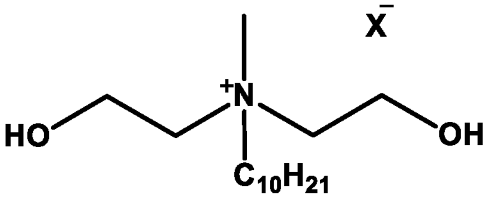
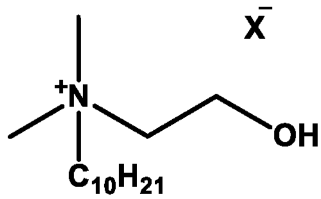
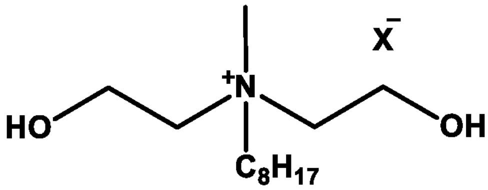
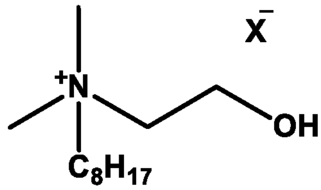

In the compounds, X is a halogen.

In an exemplary embodiment of the present specification, when a bacterial inhibition rate of the first compound is evaluated by the following Method C, the bacterial inhibition rate against *E. coli* may be 88% or more, 89.3% or more, 90% or more, 93% or more, 94.4% or more, 95% or more, 96.1% or more, 98% or more, 98.9% or more, or 99% or more, and the bacterial inhibition rate against *Proteus mirabilis* may be 83% or more, 90% or more, 91.2% or more, 95% or more, 96.9% or more, 98% or more, 99% or more, or 99.9% or more. The higher the bacterial inhibition rate, the better the antibacterial power, and the upper limit thereof is not limited, but may be, for example, 100% or less.

[Method C]

25 ml of a broth type medium (Nutrient broth, BD DIFCP., 8 g/L) inoculated with 3,000 CFU/ml bacteria is transferred to a 50-ml conical tube, 0.01 g of a first compound is added thereto, and then mixing is performed (vortexing). The well-mixed solution is cultured in a constant-temperature shaking water bath maintained at 35°C for 16 hours. After the cultured solution is diluted to 1/5 using a 1X phosphate buffered saline (PBS) buffer solution, absorbance ($\lambda$ = 600 nm) is measured using a UV/Vis spectrophotometer. The measured absorbance is compared to that of the control, and the bacterial inhibition rate is calculated by the following equation. In this case, the control means a medium solution containing no first compound.

$$\text{Bacterial inhibition rate (\%)} = \{1-(A_{sample})/(A_{reference})\} \times 100$$

($A_{sample}$: absorbance of medium solution containing first compound, $A_{reference}$: absorbance of medium solution containing no first compound)

In an exemplary embodiment of the present specification, examples of the bacteria used for the evaluation of the bacterial inhibition rate comprise *E. coli,* Proteus mirabilis, and the like.

In an exemplary embodiment of the present specification, the second compound is a compound different from the first compound.

In an exemplary embodiment of the present specification, the second compound may be a super absorbent polymer (SAP).

The super absorbent resin is a resin that can absorb moisture in an amount which is several hundreds of folds higher than its own weight and can absorb artificial urine in an amount which is several tens of folds larger than its own weight, and is a functional polymer material having excellent ability to retain water even under external pressure. These super absorbent resins are widely used in hygiene products such as diapers and sanitary napkins.

In an exemplary embodiment of the present specification, the second compound may comprise a hydrous gel polymer. In other words, the second compound is a super absorbent resin, and the super absorbent resin may comprise a hydrous gel polymer.

The hydrous gel polymer means a polymer having a moisture content of 40 wt% to 80 wt% with respect to the total weight of the hydrous gel polymer. Here, the moisture content is the content of moisture relative to the total weight of the hydrous gel polymer, and is a value obtained by subtracting the weight of the polymer in a dry state from the total weight of the hydrous gel polymer. Specifically, the moisture content is defined as a value calculated by measuring the amount of weight loss according to evaporation of moisture in the polymer during drying by increasing the temperature of the polymer through infrared heating. In this case, the moisture content is measured by increasing the temperature to about 180°C from room temperature and maintaining the temperature at 180°C under the drying conditions and setting the total drying time to 20 minutes comprising 5 minutes for which the step of increasing the temperature is performed.

The hydrous gel polymer may be prepared by cross-linking an acrylic acid-based monomer in which at least a portion of the acidic groups are neutralized and an internal cross-linking agent. For this purpose, it is possible to use a solution-state monomer composition comprising an acrylic acid-based monomer in which at least a portion of the acidic groups are neutralized, a polymerization initiator, an internal cross-linking agent and a solvent.

The acrylic acid-based monomer is a compound represented by the following Chemical Formula 2.

[Chemical Formula 2] R-COO-R'

In Chemical Formula 2, R is an alkyl group having 2 to 5 carbon atoms, which comprises an unsaturated bond, and R' is hydrogen, a monovalent or divalent metal, an ammonium group, or an organic amine salt.

Preferably, the acrylic acid-based monomer comprises one or more selected from the group consisting of acrylic acid, methacrylic acid and monovalent metal salts, divalent metal salts, ammonium salts and organic amine salts thereof.

Here, the acrylic acid-based monomer may be an acrylic acid-based monomer having an acidic group, in which at least a portion of the acidic group is neutralized. Preferably, as the monomer, it is possible to use a monomer which is partially neutralized with an alkaline material such as sodium hydroxide, potassium hydroxide, and ammonium hydroxide. In this case, the degree of neutralization of the acrylic acid-based monomer may be 40 mol% or more, or about 45 mol% or more, and 95 mol% or less, 80 mol% or less, or 75 mol% or less. The range of the degree of neutralization may be adjusted according to final physical properties. However, when the degree of neutralization is too high, neutralized monomers may be precipitated, so it may be difficult to smoothly perform polymerization, and in contrast, when the degree of neutralization is too low, the absorbency of the polymer is significantly reduced, and properties like elastic rubber that is difficult to handle may be exhibited.

The concentration of the acrylic acid-based monomer may be about 20 wt% or more, or about 40 wt% or more and about 60 wt% or less, or about 50 wt% or less with respect to a monomer composition comprising a raw material and a solvent for the super absorbent resin comprising an acrylic acid-based monomer in which at least a portion of the acid group is neutralized, a polymerization initiator, and an internal cross-linking agent, and may be an appropriate concentration in consideration of polymerization time, reaction conditions, and the like. However, when the concentration of the monomer is excessively low, the yield of the super absorbent resin may be low, and a problem with economic feasibility may be caused, and in contrast, when the concentration is excessively high, there may occur problems with process such as precipitation of some of the monomers or occurrence of a low pulverization efficiency during pulverization of a polymerized hydrous gel polymer, and physical properties of the super absorbent resin may deteriorate.

Alternatively, the internal cross-linking agent is for cross-linking the inside of the polymer obtained by polymerizing the acrylic acid-based monomer, and as a specific example thereof, it is possible to use one or more selected

among polyethylene glycol diacrylate, N,N'-methylenebisacrylamide, trimethylolpropane tri(meth)acrylate, ethylene glycol di(meth)acrylate, polyethylene glycol(meth)acrylate, propylene glycol di(meth)acrylate, polypropylene glycol(meth)acrylate, butanediol di(meth)acrylate, butylene glycol di(meth)acrylate, diethylene glycol di(meth)acrylate, hexanediol di(meth)acrylate, triethylene glycol di(meth)acrylate, tripropylene glycol di(meth)acrylate, tetraethylene glycol di(meth)acrylate, dipentaerythritol pentaacrylate, glycerin tri(meth)acrylate, pentaerythritol tetraacrylate, triarylamine, ethylene glycol diglycidyl ether, propylene glycol, glycerin, and ethylene carbonate, but the internal cross-linking agent is not limited to the above-described example.

Such an internal cross-linking agent is comprised in an amount of 0.01 to 1 part by weight with respect to 100 parts by weight of the acrylic acid-based monomer, and thus may cross-link the polymerized polymer. When the content of the internal cross-linking agent is less than 0.01 parts by weight, the improvement effect due to cross-linking is insignificant, and when the content of the internal cross-linking agent exceeds 1 part by weight, the absorption capacity of the super absorbent resin may deteriorate. More specifically, the internal cross-linking agent may be comprised in an amount of 0.01 parts by weight or more, 0.05 parts by weight or more, or 0.1 parts by weight or more, and 1 part by weight or less, 0.5 parts by weight, or 0.3 parts by weight or less with respect to 100 parts by weight of the acrylic acid-based monomer.

The polymerization initiator is not particularly limited as long as it is commonly used in the preparation of super absorbent resins.

Specifically, as the polymerization initiator, it is possible to use a thermal polymerization initiator or a photopolymerization initiator by UV irradiation depending on the polymerization method. However, even by the photopolymerization method, a certain amount of heat is generated by irradiation such as ultraviolet irradiation, and in addition, since a certain amount of heat is generated by the progress of the polymerization reaction, which is an exothermic reaction, a thermal polymerization initiator may be additionally comprised.

The photopolymerization initiator can be used without limitation in the configuration thereof as long as it is a compound capable of forming radicals by light such as ultraviolet rays.

As the photopolymerization initiator, it is possible to use, for example, one or more selected from the group consisting of benzoin ether, dialkyl acetophenone, hydroxyl alkylketone, phenyl glyoxylate, benzyl dimethyl ketal, acyl phosphine and $\alpha$-aminoketone. Meanwhile, as a specific example of acyl phosphine, it is possible to use commercially available Irgacure 819 (bis(2,4,6-trimethylbenzoyl)-phenylphosphine oxide), lucirin TPO (diphenyl(2,4,6-trimethylbenzoyl)-phosphine oxide), and the like. A wider variety of photoinitiators are well clarified in p. 115 of "UV Coatings: Basics, Recent Developments and New Application (Elsevier, 2007)", written by Reinhold Schwalm, and the photoinitiator is not limited to the above-described examples.

The photopolymerization initiator may be comprised in an amount of 0.001 parts by weight to 1 part by weight with respect to 100 parts by weight of the acrylic acid-based monomer. When the content of the photopolymerization initiator is less than 0.001 parts by weight, the polymerization rate may be slow, and when the content of the photopolymerization initiator exceeds 1 part by weight, the molecular weight of the super absorbent resin may be small, and physical properties may become non-uniform. More specifically, the photopolymerization initiator may be comprised in an amount of 0.005 parts by weight or more, 0.007 parts by weight or more, or 0.01 parts by weight or more, and 0.5 part by weight or less, 0.3 parts by weight or less, or 0.1 parts by weight or less with respect to 100 parts by weight of the acrylic acid-based monomer.

Furthermore, when a thermal polymerization initiator is further comprised as the polymerization initiator, it is possible to use one or more selected from the initiator group consisting of a persulfate-based initiator, an azo-based initiator, hydrogen peroxide and ascorbic acid as the thermal polymerization initiator. Specifically, examples of the persulfate-based initiator comprise sodium persulfate (NazSzOs), potassium persulfate ($K_2S_2O_8$), ammonium persulfate ($(NH_4)_2S_2O_8$) and the like, and examples of the azo-based initiator comprise 2,2-azobis(2-amidinopropane) dihydrochloride, 2,2-azobis-(N, N-dimethylene)isobutyramidine dihydrochloride, 2-(carbamoylazo)isobutylonitrile, 2,2-azobis[2-(2-imidazolin-2-yl)propane]dihydrochloride, 4,4-azobis-(4-cyanovalericacid), and the like. A wider variety of thermal polymerization initiators are well clarified in p. 203 of 'Principle of Polymerization(Wiley, 1981)', written by Odian, and the thermal polymerization initiator is not limited to the above-described examples.

The thermal polymerization initiator may be comprised in an amount of 0.001 parts by weight to 1 part by weight with respect to 100 parts by weight of the acrylic acid-based monomer. When the content of the thermal polymerization initiator is less than 0.001 parts by weight, additional thermal polymerization hardly occurs, so the effect of adding the thermal polymerization initiator is negligible, and when the content of the thermal polymerization initiator exceeds 1 part by weight, the molecular weight of the super absorbent resin may be small, and physical properties may become non-uniform. More specifically, the thermal polymerization initiator may be comprised in an amount of 0.005 parts by weight or more, or 0.01 parts by weight or more, or 0.1 parts by weight or more, and 0.5 parts by weight or less, or 0.3 parts by weight or less with respect to 100 parts by weight of the acrylic acid-based monomer.

In addition to the polymerization initiator, one or more additive such as a surfactant, a thickener, a plasticizer, a preservation stabilizer, and an antioxidant may be added as necessary during cross-linking polymerization.

A monomer composition comprising the above-described acrylic acid-based monomer, internal cross-linking agent, polymerization initiator, and optionally an additive may be prepared in the form of a solution dissolved in a solvent.

The solvent that can be used in this case can be used without limitation in the composition thereof as long as it can dissolve the above-described components, and for example, it is possible to use a combination of one or more selected among water, ethanol, ethylene glycol, diethylene glycol, triethylene glycol, 1,4-butanediol, propylene glycol, ethylene glycol monobutyl ether, propylene glycol monomethyl ether, propylene glycol monomethyl ether acetate, methyl ethyl ketone, acetone, methyl amyl ketone, cyclohexanone, cyclopentanone, diethylene glycol monomethyl ether, diethylene glycol ethyl ether, toluene, xylene, butyrolactone, carbitol, methyl cellosolve acetate, N, N-dimethylacetamide, and the like, but the solvent is not limited to the above-described examples. The solvent may be comprised in the residual amount of the monomer composition except for the above-described components with respect to the total content of the monomer composition.

Meanwhile, the method of forming a hydrous gel polymer by photopolymerizing such a monomer composition is not particularly limited in configuration as long as it is a typically used polymerization method.

Specifically, the photopolymerization may be performed by irradiating the monomer composition with an ultraviolet ray having an intensity of 5 mW or more, 8 mW or more, or 10 mW or more and 30 mW or less, or 20 mW or lesss at a temperature of 60°C or more, or 70°C or more, and 90°C or less, or 85°C or less. Under the aforementioned conditions, a cross-linked polymer can be formed with a polymerization efficiency which is better than that during photopolymerization.

Alternatively, when the photopolymerization is performed, the photopolymerization may be performed in a reactor equipped with a movable conveyor belt, but the above-described polymerization method is an example, and the present invention is not limited to the above-described polymerization method.

Furthermore, when the photopolymerization is performed in a reactor equipped with a movable conveyor belt as described above, the form of a hydrous gel polymer typically obtained may be a sheet-like hydrous gel polymer having the width of the belt. In this case, the thickness of the polymer sheet varies depending on the concentration of the monomer composition to be injected and the injection rate, but it is desirable to feed the monomer composition such that a sheet-like polymer having a thickness of about 0.5 cm to about 5 cm can be obtained. When the monomer composition is fed so that the thickness of the sheet-like polymer becomes too thin, the production efficiency becomes low, which is not preferred, and when the thickness of the sheet-like polymer exceeds 5 cm, the polymerization reaction may not uniformly occur throughout the entire thickness due to the excessively high thickness.

The hydrous gel polymer polymerized as described above may finally take on a particle form through drying, pulverization, and classification processes.

The drying method can be used without limitation as long as it is a method typically used as a drying process for a hydrous gel polymer. Specifically, the drying step may be performed by methods such as hot air supply, irradiation with infrared rays, irradiation with ultrahigh frequency waves or irradiation with ultraviolet rays.

The moisture content of the hydrous gel polymer after drying may be 1 wt% to 10 wt%, 1 wt% to 5 wt%.

The pulverization step may be performed such that the particle diameter of the hydrous gel polymer is 150 μm to 850 μm. Specifically, the polymer may be pulverized using a pulverizer such as a pin mill, a hammer mill, a screw mill, a roll mill, a disc mill or a jog mill, but the pulverizer is not limited thereto.

After the pulverization process, a process of classifying the hydrous gel polymer according to particle diameter may be performed.

In the antibacterial deodorant composition according to an exemplary embodiment of the present specification, the first compound is cross-linked to at least a portion of the second compound.

In an exemplary embodiment of the present specification, the antibacterial deodorant composition may be present in the form of particles having a sea-island structure. Specifically, the second compound constitutes a core structure, the first compound is cross-linked to at least a portion of the second compound to form a discontinuous cross-linked structure in the form of islands on the core, and FIG. 1 illustrates the particle structure of the antibacterial deodorant composition of the present invention.

In the related art, antibacterial agents were introduced in the form of additives in order to impart antibacterial properties to super absorbent resins, but the safety of the super absorbent resin deteriorates, or basic physical properties such as absorbency deteriorate, and there is a problem in the durability of antibacterial properties, and the like.

However, as in the present invention, when the first compound having antibacterial and deodorant properties is present in a cross-linked form on the surface of the second compound, it is possible to have the antibacterial and deodorant effects while maintaining the absorbency.

In an exemplary embodiment of the present specification, the antibacterial deodorant composition may comprise the first compound in an amount of 0.4 parts by weight or more and 2.5 parts by weight or less, or 0.5 parts by weight or more and 2 parts by weight or less based on 100 parts by weight of the entire second compound.

When the first compound is comprised within the above range, the first compound may be appropriately cross-linked to the surface of the second compound to expect excellent antibacterial and deodorant effect while having sufficient

absorption capacity and retention capacity.

There are problems in that when the first compound is comprised in an amount of less than 0.4 parts by weight, antibacterial and deodorant effects are insignificant, and when the first compound is comprised in an amount of more than 2.5 parts by weight, absorption capacity and retention capacity deteriorate.

In an exemplary embodiment of the present specification, the antibacterial deodorant composition may have an antibacterial power of 90% or more during an antibacterial evaluation.

In an exemplary embodiment of the present specification, the antibacterial deodorant composition may have an antibacterial power of 90% or more, 90.5% or more, 91.1% or more or 92.1% or more, preferably 95% or more, 97% or more, or 97.5% or more, and more preferably 99% or more, or 99.2% or more during the antibacterial evaluation. The higher antibacterial power means that the antibacterial power is better, and the upper limit thereof is not limited, but may be, for example, 100% or less.

In an exemplary embodiment of the present specification, the antibacterial deodorant composition may have an antibacterial power against *E. coli* of 90% or more, or 93% or more, preferably 95% or more, and more preferably 97% or more, or 99% or more during the antibacterial evaluation.

In an exemplary embodiment of the present specification, the antibacterial deodorant composition may have an antibacterial power against Proteus mirabilis of 90% or more, 90.5% or more, 91.1% or more or 92.1% or more, preferably 95% or more, 97% or more, or 97.5% or more, and more preferably 99% or more, or 99.2% or more during the antibacterial evaluation.

In an exemplary embodiment of the present specification, the antibacterial evaluation may be determined by the following Method B.

[Method B]

After 40 ml of artificial urine inoculated with 3,000 CFU/ml bacteria is poured into 2 g of the antibacterial deodorant composition, the resulting mixture is cultured at 35°C for 12 hours, and after the cultured solution is diluted with 160 ml of a physiological saline solution, samples serially diluted with the physiological saline solution are spread on an agar plate for calculation.

Specifically, 100 μm of the diluted sample was dropped on the agar plate and incubated at 30°C for about 24 hours, and then the number of bacteria was counted, and the antibacterial power was calculated and evaluated by the following equation from the number of bacteria and the number of bacteria of the control not surface-treated with the first compound (antibacterial deodorant monomer). Here, the control means a composition not surface-treated with the first compound.

$$\text{Antibacterial power (\%)} = \{1-(N_{sample})/(N_{reference})\} \times 100$$

($N_{sample}$: number of bacteria of sample containing first compound, $N_{reference}$: number of bacteria of control containing no first compound)

In an exemplary embodiment of the present specification, the bacteria used in the antibacterial evaluation may be at least one of Gram-positive bacteria and Gram-negative bacteria.

In an exemplary embodiment of the present specification, the bacteria used in the antibacterial evaluation may be at least one of Proteus mirabilis, E. coli, E. cloacae and E. faecalis.

In an exemplary embodiment of the present specification, the bacteria used in the antibacterial evaluation may be Proteus mirabilis, or E. coli.

In an exemplary embodiment of the present specification, the ammonia content of the antibacterial deodorant composition may be 190 ppm or less during an ammonia deodorization evaluation.

In an exemplary embodiment of the present specification, the antibacterial deodorant composition may have an ammonia content of 190 ppm or less, 180 ppm or less, 160 ppm or less, or 150 ppm or less, preferably 100 ppm or less, 80 ppm or less, 60 ppm or less, or 50 ppm or less, and more preferably 20 ppm or less, or 10 ppm or less during the ammonia deodorization evaluation, and the lower limit thereof is not limited, but may be, for example, 0 ppm or more. The smaller ammonia content means that the deodorizing power against ammonia is better.

In an exemplary embodiment of the present specification, the ammonia deodorization evaluation may be determined by the following Method D.

[Method D]

After 40 ml of artificial urine inoculated with 3,000 CFU/ml bacteria is poured into 2 g of the antibacterial deodorant composition, the resulting mixture is cultured at 35°C for 12 hours, and an ammonia content is analyzed and

measured by allowing the cultured solution to pass through an ammonia detector tube.

In an exemplary embodiment of the present specification, a 3M ammonia detector tube may be used as the ammonia detector tube.

In an exemplary embodiment of the present specification, the bacteria used in the ammonia deodorization evaluation may be at least one of Gram-positive bacteria and Gram-negative bacteria.

In an exemplary embodiment of the present specification, the bacteria used in the ammonia deodorization evaluation may be at least one of Proteus mirabilis, E. coli, E. cloacae and E. faecalis.

In an exemplary embodiment of the present specification, the bacteria used in the ammonia deodorization evaluation may be Proteus mirabilis, or E. coli.

In an exemplary embodiment of the present specification, the antibacterial deodorant composition may have a centrifuge retention capacity (CRC) of 30 g/g or more and 60 g/g or less.

In an exemplary embodiment of the present specification, the antibacterial deodorant composition may have a centrifuge retention capacity of 30 g/g or more, 32 g/g or more, 34 g/g or more, 36 g/g or more, or 37 g/g or more. Further, the antibacterial deodorant composition may have a centrifuge retention capacity of 60 g/g or less, 55 g/g or less, 50 g/g or less, 48 g/g or less, 45 g/g or less, 43 g/g or less, or 42 g/g or less.

When the antibacterial deodorant composition having a centrifuge retention capacity within the above range is used for diapers or sanitary napkins, the diapers or sanitary napkins can absorb water well even in a standing position.

In an exemplary embodiment of the present specification, the centrifuge retention capacity (CRC) may be measured in accordance with EDANA WSP 241.3. Specifically, $W_0$ (g) of the antibacterial deodorant composition of the present invention is uniformly placed in a non-woven fabric bag, the bag was sealed, and then the sealed bag is immersed in a physiological saline solution at room temperature. After 30 minutes have passed, moisture is removed from the bag for 3 minutes under a condition of 250 G using a centrifuge, and the mass $W_2$ (g) of the bag is measured. Alternatively, the mass $W_1$ (g) is measured after performing the experiment in the same manner as described above without using the antibacterial deodorant composition. Using each obtained mass, the centrifuge retention capacity (CRC) (g/g) is calculated according to the following equation.

[Equation 1]

$$\text{CRC (g/g)} = \{[W_2\,(g) - W_1\,(g)]/W_0\,(g)\} - 1$$

In an exemplary embodiment of the present specification, the antibacterial deodorant composition may have an absorbing under pressure (AUP) of 10 g/g or more and 40 g/g or less.

In an exemplary embodiment of the present specification, the antibacterial deodorant composition may have an absorbing under pressure of 10 g/g or more, 14 g/g or more, 18 g/g or more, 20 g/g or more, or 21 g/g or more. In addition, the antibacterial deodorant composition may have an absorbing under pressure of 40 g/g or less, 38 g/g or less, 35 g/g or less, 32 g/g or less, 30 g/g or less, 28 g/g or less, or 27 g/g or less.

When the antibacterial deodorant composition having an absorbing under pressure within the above range is used for diapers or sanitary napkins, water does not leak again even when one is sitting or lying down.

In an exemplary embodiment of the present specification, an absorbing under pressure (AUP) of 0.5 psi to 0.8 psi may be measured in accordance with EDANA WSP 242.3. Specifically, a 400-mesh stainless steel wire mesh is mounted to the bottom of a plastic cylinder having an inner diameter of 60 mm. $W_0$ (g) of the antibacterial deodorant composition of the present invention is uniformly sprayed on a wire mesh under conditions of room temperature and humidity of 50%, and a piston capable of further applying a load of 0.5 psi to 0.8 psi uniformly thereon is slightly smaller than an outer diameter of 60 mm and has no gaps with the inner wall of the cylinder, and the vertical movement thereof is not hindered. In this case, the weight $W_3$ (g) of the device is measured. A glass filter having a diameter of 90 mm and a thickness of 5 mm is placed inside a petri dish having a diameter of 150 mm and a physiological saline solution composed of 0.9 wt% sodium chloride is brought flush with the top surface of the glass filter. A sheet of filter paper with a diameter of 90 mm is placed thereon. The measuring device is placed on the filter paper and allows a liquid to be absorbed under load for 1 hour. After 1 hour, the measuring device is lifted and its weight $W_4$ (g) is measured. Using each obtained mass, the absorbing under pressure (AUP)(g/g) is calculated according to the following equation.

[Equation 2]

$$\text{AUP (g/g)} = [W_4\,(g) - W_3\,(g)]/W_0\,(g)$$

An exemplary embodiment of the present specification provides a method for preparing an antibacterial deodorant composition.

Specifically, the method for preparing an antibacterial deodorant composition according to an exemplary embodiment of the present specification comprises: (a) preparing a mixture of the first compound of Chemical Formula 1 and a second compound different from the first compound; and (b) cross-linking the mixture.

In an exemplary embodiment of the present specification, Step (b) may be performed at 150°C to 220°C for a period of time of more than 20 minutes, and may be performed at 170°C to 200°C for a period of time of 30 minutes to 80 minutes.

When the mixture is cross-linked for 20 minutes or less in Step (b), the first compound is not sufficiently cross-linked on the surface of the second compound, so there are problems in that the desired antibacterial power and deodorizing power cannot be obtained and absorption ability deteriorates.

In an exemplary embodiment of the present specification, in Step (b), the mixture may be cross-linked for more than 20 minutes, more than 20 minutes and 80 minutes or less, preferably 50 minutes or more and 80 minutes or less. When the first compound and the second compound are cross-linked for a time within the above range, optimized retention capacity and absorption capacity may be obtained, and antibacterial and deodorizing functions may also be obtained. When the first compound and the second compound are cross-linked for more than 80 minutes, there is a problem in that the retention capacity is gradually reduced.

In an exemplary embodiment of the present specification, in Step (b), the mixture may be cross-linked at 150°C to 220°C, or 170°C to 200°C.

In an exemplary embodiment of the present specification, Step (a) of the preparing of the mixture of the first compound and the second compound may comprise (a1) preparing a first compound of Chemical Formula 1 and a second compound different from the first compound and (a2) mixing the first compound and the second compound.

In an exemplary embodiment of the present specification, the first compound and the second compound may be directly prepared, or commercially available products may be used.

In an exemplary embodiment of the present specification, the mixing method is not limited as long as it can uniformly mix the first compound and the second compound.

In an exemplary embodiment of the present specification, the mixture may further comprise a surface cross-linking agent. The surface cross-linking agent forms a cross-linking bond on the surface of the second compound separately from the first compound, and the absorption capacity may be improved by comprising the surface cross-linking agent.

In an exemplary embodiment of the present specification, as the surface cross-linking agent, it is possible to use one or more selected from the group consisting of a polyhydric alcohol-based compound; an epoxy compound; a polyamine compound; a haloepoxy compound; a condensation product of the haloepoxy compound; an oxazoline compound; a mono-, di-, or poly-oxazolidinone compound; a cyclic urea compound; a polyvalent metal salt; and an alkylene carbonate-based compound. Preferably, an alkylene carbonate-based compound may be used.

In an exemplary embodiment of the present specification, the surface cross-linking agent may comprise one or more of ethylene carbonate, propylene carbonate, and the like.

In an exemplary embodiment of the present specification, the surface cross-linking agent may be comprised in an amount of 0.01 parts by weight or more and 4 parts by weight or less, 0.05 parts by weight or more and 3 parts by weight or less, or 0.1 parts by weight or more and 2.5 parts by weight or less based on 100 parts by weight of the entire second compound.

In an exemplary embodiment of the present specification, the mixture may further comprise an ionic cross-linking agent. The ionic cross-linking agent serves to increase the efficiency of surface cross-linking between the first compound and the second compound.

In an exemplary embodiment of the present specification, as the ionic cross-linking agent, it is possible to use one or more selected among $Al_2(SO4)_3$, $AlO_3$, $Al_2O_3 \cdot 3SiO_2$, and $Al(H_2O)_6^{3+}$, but the ionic cross-linking agent is not limited thereto. Preferably, $Al_2(SO_4)_3$ (aluminum sulfate) may be used.

In an exemplary embodiment of the present specification, the ionic cross-linking agent may be comprised in an amount of 0.01 parts by weight or more and 3 parts by weight or less, 0.05 parts by weight or more and 2 parts by weight or less, or 0.1 parts by weight or more and 1.5 parts by weight or less based on 100 parts by weight of the entire second compound.

In an exemplary embodiment of the present specification, the mixture may further comprise a surfactant. When the surfactant is comprised, there is an effect of imparting weak hydrophobicity to water to prevent excessive absorption of water while the super absorbent resin is surface-treated.

In an exemplary embodiment of the present specification, as the surfactant, it is possible to use one or more selected among a polycarboxylate-based surfactant; and a polyethylene glycol-based surfactant. Preferably, a polycarboxylate-based surfactant may be used.

In an exemplary embodiment of the present specification, the surfactant may be comprised in an amount of 0.005 parts by weight or more and 0.5 parts by weight or less, 0.01 parts by weight or more and 0.3 parts by weight or

less, 0.03 parts by weight or more and 0.15 parts by weight or less, or 0.05 parts by weight or more and 0.1 parts by weight or less based on 100 parts by weight of the entire second compound.

In an exemplary embodiment of the present specification, the mixture may further comprise water or alcohol. By comprising water or alcohol, the mixture is prepared in the form of a solution, and the first compound may be evenly dispersed in the second compound.

In an exemplary embodiment of the present specification, the water or alcohol may be comprised in an amount of 1 part by weight or more and 20 parts by weight or less, 2 parts by weight or more and 15 parts by weight or less, or 3 parts by weight or more and 10 parts by weight or less based on 100 parts by weight of the entire second compound.

In an exemplary embodiment of the present specification, Step (c) of classifying the antibacterial deodorant composition may be additionally comprised after Step (b).

The classification process may use a standard mesh of ASTM standard.

The antibacterial deodorant composition according to an exemplary embodiment of the present specification may be used for super absorbent antibacterial products.

Examples of the super absorbent antibacterial products comprise diapers, sanitary napkins, and the like, but are not limited thereto.

An exemplary embodiment of the present specification provides a deodorant composition comprising a first compound of the following Chemical Formula 1.

[Chemical Formula 1]

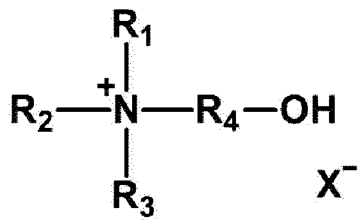

In Chemical Formula 1,

$R_1$ to $R_3$ are each independently an alkyl group having 1 to 12 carbon atoms, which is unsubstituted or substituted with a hydroxyl group,
at least one of $R_1$ to $R_3$ is an alkyl group having 8 to 12 carbon atoms, and $R_4$ is an alkylene group having 1 to 6 carbon atoms, and
X is a halogen.

The description on the first compound of Chemical Formula 1 of the above-described antibacterial deodorant composition may be equally applied to the first compound of Chemical Formula 1 of the deodorant composition.

In an exemplary embodiment of the present specification, the deodorant composition has a bacterial inhibition rate of 80% or more against at least one strain of Gram-positive bacteria and Gram-negative bacteria as evaluated by the following Method E.

[Method E]

25 ml of a broth type medium (Nutrient broth, BD DIFCP., 8 g/L) inoculated with 3,000 CFU/ml bacteria is transferred to a 50-ml conical tube, 0.01 g of the deodorant composition is added thereto, and then mixing is performed (vortexing). The well-mixed solution is cultured in a constant-temperature shaking water bath maintained at 35°C for 16 hours. After the cultured solution is diluted to 1/5 using a 1X phosphate buffered saline (PBS) buffer solution, absorbance ($\lambda$ = 600 nm) is measured using a UV/Vis spectrophotometer. The measured absorbance is compared to that of the control, and the bacterial inhibition rate is calculated by the following equation. In this case, the control means a medium solution which does not contain the deodorant composition.

$$\text{Bacterial inhibition rate (\%)} = \{1-(A_{sample})/(A_{reference})\} \times 100$$

($A_{sample}$: absorbance of medium solution containing deodorant composition, $A_{reference}$: absorbance of medium solution containing no deodorant composition)

In an exemplary embodiment of the present specification, when a bacterial inhibition rate of the deodorant composition is evaluated by the following Method E, the bacterial inhibition rate against *E. coli* may be 88% or more, 89.3% or more, 90% or more, 93% or more, 94.4% or more, 95% or more, 96.1% or more, 98% or more, 98.9% or more, or 99% or more, and the bacterial inhibition rate against *Proteus mirabilis* may be 83% or more, 90% or more, 91.2% or more, 95% or more, 96.9% or more, 98% or more, 99% or more, or 99.9% or more. The higher the bacterial inhibition rate, the better the antibacterial power, and the upper limit thereof is not limited, but may be, for example, 100% or less.

[Mode for Invention]

Hereinafter, the present specification will be described in detail with reference to Examples for specifically describing the present specification. However, the Examples according to the present specification may be modified in various forms, and it is not interpreted that the scope of the present specification is limited to the Examples described below. The Examples of the present specification are provided to more completely explain the present specification to a person with ordinary skill in the art.

**<Preparation Example 1> Preparation of first compound (antibacterial deodorant monomer)**

Preparation Example A

After 130 ml of ethanol, 0.131 mol of methyldiethanolamine, and 0.144 mol of bromodecane were put into a 250 ml flask, a reaction was performed by stirring the mixture at 65°C for 24 hours using a magnetic bar. After 24 hours, the completely reacted solution was added to 800 ml of a diethyl ether solution to obtain a precipitate, and then the reaction product was filtered using a vacuum filter, and the remaining diethyl ether in a vacuum oven was completely removed to obtain Antibacterial Deodorant Monomer A, which is a final synthetic product.

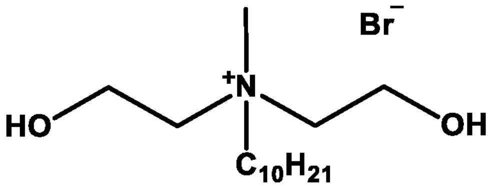

Preparation Example B

Antibacterial Deodorant Monomer B was obtained by performing the preparation in the same manner as in Preparation Example A, except that bromooctane was used instead of bromodecane in the preparation method of Preparation Example A.

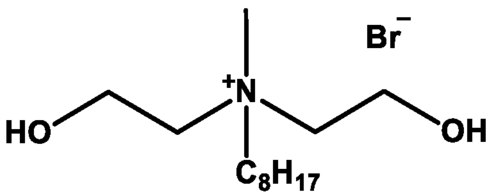

Preparation Example C

Antibacterial Deodorant Monomer C was obtained by performing the preparation in the same manner as in Preparation Example A, except that bromododecane was used instead of bromodecane in the preparation method of Preparation Example A.

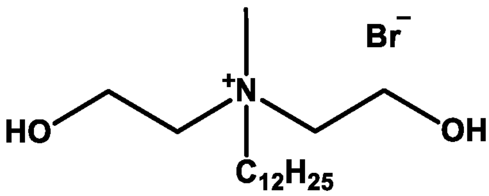

Preparation Example D

Antibacterial Deodorant Monomer D was obtained by performing the preparation in the same manner as in Preparation Example A, except that dimethylethanolamine was used instead of methyldiethanolamine in the preparation method of Preparation Example A.

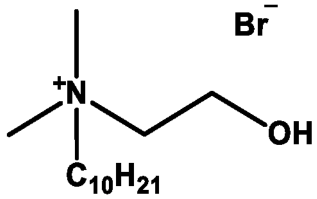

Preparation Example E

Antibacterial Deodorant Monomer E was obtained by performing the preparation in the same manner as in Preparation Example A, except that dimethylethanolamine and bromododecane were used instead of methyldiethanolamine and bromodecane, respectively, in the preparation method of Preparation Example A.

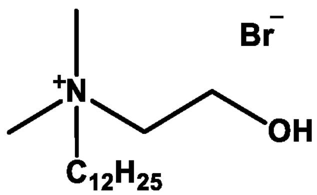

Preparation Example F

Antibacterial Deodorant Monomer F was obtained by performing the preparation in the same manner as in Preparation Example A, except that dimethylethanolamine and chlorodecane were used instead of methyldiethanolamine and bromodecane, respectively, in the preparation method of Preparation Example A.

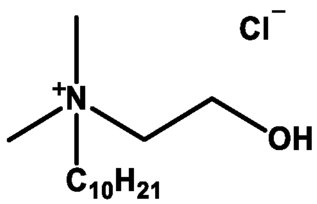

Preparation Example G

Antibacterial Deodorant Monomer G was obtained by performing the preparation in the same manner as in Preparation Example A, except that bromobutane was used instead of bromodecane in the preparation method of Preparation Example A.

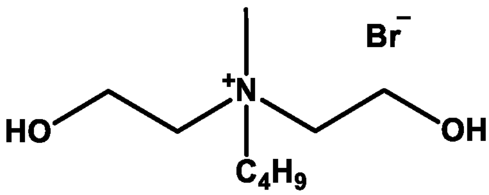

The NMR data of Antibacterial Deodorant Monomers A to G prepared in Preparation Example 1 can be confirmed in FIGS. 2 to 8.

FIG. 2 illustrates the NMR data of Antibacterial Deodorant Monomer A.

FIG. 3 illustrates the NMR data of Antibacterial Deodorant Monomer B.

FIG. 4 illustrates the NMR data of Antibacterial Deodorant Monomer C.

FIG. 5 illustrates the NMR data of Antibacterial Deodorant Monomer D.

FIG. 6 illustrates the NMR data of Antibacterial Deodorant Monomer E.

FIG. 7 illustrates the NMR data of Antibacterial Deodorant Monomer F.

FIG. 8 illustrates the NMR data of Antibacterial Deodorant Monomer G.

**<Preparation Example 2> Preparation of second compound (Super Absorbent Resin 1)**

A photoinitiator bis(2,4,6-trimethylbenzoyl)-phenylphosphineoxide (I-819) was dissolved in acrylic acid to prepare a 0.21 wt% solution, and polyethylene glycol diacrylate (PEGDA, Mw = 523) used as a cross-linking agent was dissolved in acrylic acid to prepare a 20 wt% PEGDA solution. Then, a thermal initiator sodium persulfate (SPS) was dissolved in water to prepare a 4 wt% aqueous solution. Next, 634.6 g of 31.5 wt% caustic soda (NaOH) and 234.7 g of water were put into a Buchi reactor and diluted, and 489.7 g of acrylic acid, 5.91 g of 20 wt% PEGDA, and 19.58 g of 0.21 wt% I-819 were put into another Buchi reactor and mixed. 15.42 g of 4 wt% SPS was put into a 2 L flask beaker. The well-diluted caustic soda was transferred to the Buchi reactor containing acrylic acid, the acrylic acid was neutralized, and when the temperature of the neutralized solution reached 43°C, the neutralized solution was transferred to a 2 L flask beaker containing SPS. When the temperature of the neutralized solution even mixed with SPS reached 40°C, the neutralized solution was put into a tray in a UV chamber. Thereafter, while maintaining the temperature of the polymerization atmosphere at 80°C, the tray was irradiated with ultraviolet rays for 1 minute using a UV irradiation device (irradiation amount: 10 $mW/cm^2$), and UV polymerization was performed by aging for 2 minutes to produce a hydrous gel polymer sheet. After the polymerized sheet was taken out and cut into a size of 3 cm x 3 cm, 180 g of water was added thereto, the resulting mixture was mixed, and then chopping was performed using a meat chopper to produce a crumb. In this case, the meat chopper had a hole size of 16 pi. The crumb was dried in an oven capable of up-and-down air volume transfer. Hot air at 185°C was flowed from bottom to top for 16 minutes and from top to bottom for 16 minutes to uniformly dry the crumb, and the moisture content of a dried body after drying was allowed to be 2% or less. After drying, the dried body was pulverized with a pulverizer, classified for 10 minutes with Amplitude 1.5 mm (classification mesh combination: #20-30/#30-50/#50-100/#100), each classified fraction (22%/64%/13%/1%) was collected to obtain a polymer with a particle diameter of about 850 μm or less by classification, and a base resin powder was obtained in this manner.

**<Preparation Example 3> Preparation of second compound (Super Absorbent Resin 2)**

An experiment was performed in the same manner as in Preparation Example 2, except that the following antibacterial deodorant monomer was uniformly diluted by weighing 1 to 2 phr of the following antibacterial deodorant monomer relative to the weight of acrylic acid and introduced into the UV chamber before adding the neutralizing solution to the UV chamber in Preparation Example 2

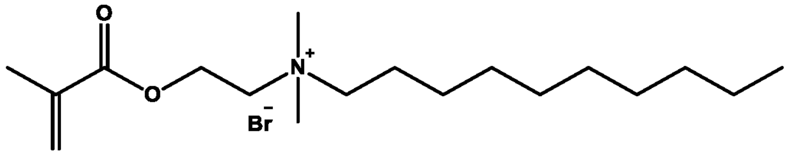

**<Preparation Example 4> Preparation of antibacterial deodorant composition**

Example 1

A surface cross-linking solution comprising 4.4 parts by weight of water, 0.3 parts by weight of ethylene carbonate, 0.075 parts by weight of a polycarboxylate surfactant, 0.3 parts by weight of aluminum sulfate, and 0.5 parts by weight of Antibacterial Deodorant Monomer A was sprayed onto 100 parts by weight of Super Absorbent Resin 1 prepared in Preparation Example 2 and mixed, and a surface cross-linking reaction was performed at 180°C for 70 minutes by putting the resulting mixture into a container composed of a stirrer and a double jacket. Thereafter, the surface-treated powder was classified with a standard mesh of ASTM standard to obtain an antibacterial deodorant composition comprising particles with a size of 150 μm to 850 μm.

Examples 2 to 6 and Comparative Examples 1 to 7

An antibacterial deodorant composition was obtained by performing the preparation in the same manner as in Example 1, except that the first compound of the type shown in the following Table 1 was used instead of Antibacterial Deodorant Monomer A in Example 1, and the surface cross-linking reaction was performed for the time shown in the following Table 1.

Comparative Example 8

Super Absorbent Resin 2 prepared in Preparation Example 3 was used as an antibacterial deodorant composition.

[Table 1]

| Antibacterial deodorant composition | First compound | Second compound | Time for surface cross-linking reaction |
|---|---|---|---|
| Example 1 | Antibacterial Deodorant Monomer A | Super Absorbent Resin 1 | 70 min |
| Example 2 | Antibacterial Deodorant Monomer B | | |
| Example 3 | Antibacterial Deodorant Monomer C | | |
| Example 4 | Antibacterial Deodorant Monomer D | | |
| Example 5 | Antibacterial Deodorant Monomer E | | |
| Example 6 | Antibacterial Deodorant Monomer F | | |
| Comparative Example 1 | Antibacterial Deodorant Monomer G | | |
| Comparative Example 2 | Guanidine | | |
| Comparative Example 3 | Hexadecyltrimethylammonium chloride | | |
| Comparative Example 4 | Didodecyldimethylammonium chloride | | |
| Comparative Example 5 | 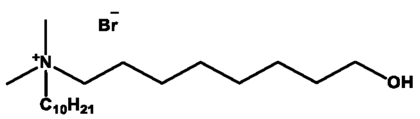 | | |
| Comparative Example 6 | 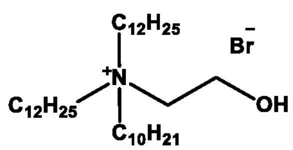 | | |
| Comparative Example 7 | Antibacterial Deodorant Monomer A | | 20 min |
| Comparative Example 8 | Super Absorbent Resin 2 | | 70 min |

Example 7

A surface cross-linking solution comprising 9.46 parts by weight of water, 1.2 parts by weight of ethylene carbonate, 1.2 parts by weight of propylene carbonate, 1.14 parts by weight of an aqueous aluminum sulfate solution, and 1 part by weight of Antibacterial Deodorant Monomer A was sprayed onto 100 parts by weight of Super Absorbent Resin 1 prepared in Preparation Example 2 and mixed, and a surface cross-linking reaction was performed at 190°C for 30 minutes by putting the resulting mixture into a container composed of a stirrer and a double jacket. Thereafter, the surface-treated powder was classified with a standard mesh of ASTM standard to obtain an antibacterial deodorant composition comprising particles with a size of 150 μm to 850 μm.

Examples 8 to 13 and Comparative Example 9

An antibacterial deodorant composition was obtained by performing the preparation in the same manner as in Example 7, except that a first compound of the type and content shown in the following Table 2 was used instead of 1 part by weight of Antibacterial Deodorant Monomer A in Example 7.

Comparative Example 10

Super Absorbent Resin 1 prepared in Preparation Example 1 was used as an antibacterial deodorant composition.

[Table 2]

| | First compound | | Second compound |
|---|---|---|---|
| | Type | Content | |
| Example 7 | Antibacterial Deodorant Monomer A | 1 part by weight | Super Absorbent Resin 1 |
| Example 8 | Antibacterial Deodorant Monomer A | 1.5 part by weight | Super Absorbent Resin 1 |
| Example 9 | Antibacterial Deodorant Monomer A | 2 part by weight | Super Absorbent Resin 1 |
| Example 10 | Antibacterial Deodorant Monomer C | 0.5 part by weight | Super Absorbent Resin 1 |
| Example 11 | Antibacterial Deodorant Monomer C | 1 part by weight | Super Absorbent Resin 1 |
| Example 12 | Antibacterial Deodorant Monomer C | 1.5 part by weight | Super Absorbent Resin 1 |
| Example 13 | Antibacterial Deodorant Monomer C | 2 part by weight | Super Absorbent Resin 1 |
| Comparative Example 9 | Antibacterial Deodorant Monomer A | 0.3 part by weight | Super Absorbent Resin 1 |
| Comparative Example 10 | - | - | Super Absorbent Resin 1 |

**<Experimental Example 1> Antibacterial power measurement of first compound (antibacterial deodorant monomer)**

25 ml of a broth type medium (Nutrient broth, BD DIFCP., 8 g/L) inoculated with 3,000 CFU/ml E. coli (or Proteus mirabilis) was transferred to a 50-ml conical tube, 0.01 g of the first compound (antibacterial deodorant monomer) in Table 1 was added thereto, and then mixing was performed (vortexing). The well-mixed solution was cultured in a constant-temperature shaking water bath maintained at 35°C for 16 hours. After the cultured solution was diluted to 1/5 using a 1X phosphate buffered saline (PBS) buffer solution, absorbance (λ = 600 nm) was measured using a UV/Vis spectrophotometer. The measured absorbance was compared with a solution cultured without the first compound (antibacterial deodorant monomer), and the bacteria inhibition rate was calculated using the following equation, and is shown in the following Table 3.

$$\text{Bacterial inhibition rate (\%)} = \{1-(A_{sample})/(A_{reference})\} \times 100$$

($A_{sample}$: absorbance of medium solution containing antibacterial deodorant monomer, $A_{reference}$: absorbance of medium solution containing no antibacterial deodorant monomer)

[Table 3]

| | Bacterial inhibition rate (%) | |
|---|---|---|
| | E. Coli | Proteus mirabilis |
| Antibacterial Deodorant Monomer A | 98.9 | 99.9 |
| Antibacterial Deodorant Monomer B | 89.3 | 91.2 |
| Antibacterial Deodorant Monomer C | 99.1 | 99.9 |
| Antibacterial Deodorant Monomer D | 94.4 | 91.7 |
| Antibacterial Deodorant Monomer E | 96.1 | 96.9 |
| Antibacterial Deodorant Monomer F | 96.4 | 83.1 |
| Antibacterial Deodorant Monomer G | 15.2 | 18.3 |
| Guanidine | 96.5 | 97.2 |
| Hexadecyltrimethylammonium chloride | 92.1 | 93.5 |
| Didodecyldimethylammonium chloride | 90.1 | 88.5 |
| 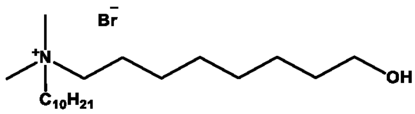 | 82.1 | 85.1 |
| 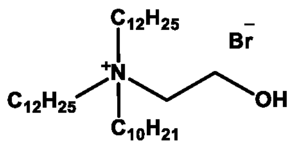 | 79.8 | 81.3 |

**<Experimental Example 2> Deodorizing power measurement of antibacterial deodorant composition**

After 1 g of the antibacterial deodorant composition was put into a 500-ml Lab bottle, 25 ml of artificial urine inoculated with $10^5$ CFU/mL E. coli, $10^6$ CFU/mL Proteus mirabilis and $10^6$ CFU/mL E. cloacae was injected, and the resulting mixture was cultured. After the mixture was cultured at 35°C for 24 hours, diacetyl, 3-methylbutanol, DMDS+DMTS, guaiacol, and p-cresol, which are the odorous components of artificial urine, were captured in adsorption tubes, the mass of each captured component was analyzed using GC/MS, and the deodorization evaluation results measured above are shown in the following Table 4.

In the following Table 4, Reference is data using a super absorbent resin that has not been subjected to surface cross-linking treatment.

[Table 4]

| | Diacetyl | 3-methylbutanol | DMDS+DMTS | Guaiacol | p-Cresol |
|---|---|---|---|---|---|
| Reference | 95 | 685 | <20 | 1030 | <10 |
| Example 1 | 25 | 130 | <20 | 170 | <10 |
| Example 2 | 28 | 205 | <20 | 251 | <10 |
| Example 3 | 20 | 102 | <20 | 172 | <10 |
| Example 4 | 24 | 129 | <20 | 173 | <10 |
| Example 5 | 21 | 99 | <20 | 199 | <10 |
| Example 6 | 25 | 124 | <20 | 168 | <10 |
| Comparative Example 1 | 81 | 582 | <20 | 572 | <10 |
| Comparative Example 2 | 41 | 168 | <20 | 207 | <10 |

(continued)

| | Diacetyl | 3-methylbutanol | DMDS+DMTS | Guaiacol | p-Cresol |
|---|---|---|---|---|---|
| Comparative Example 3 | 50 | 247 | <20 | 310 | <10 |
| Comparative Example 4 | 53 | 250 | <20 | 318 | <10 |
| Comparative Example 5 | 36 | 149 | <20 | 215 | <10 |
| Comparative Example 6 | 34 | 156 | <20 | 198 | <10 |
| Comparative Example 7 | 81 | 601 | <20 | 988 | <10 |
| Comparative Example 8 | 35 | 145 | <20 | 194 | <10 |

Through Table 4, it can be seen that when a compound (Comparative Example 1) in which the number of carbon atoms in the alkyl group $R_1$ is smaller than that of the compound of the present invention, a compound (Comparative Example 2) different from the present invention, or a compound comprising no hydroxyl group (Comparative Example 3 or 4) was used, or the antibacterial deodorant monomer was comprised during preparation of the super absorbent resin (Comparative Example 8), the deodorizing power was reduced. In addition, in Comparative Example 7, the surface cross-linking reaction time was shorter than that of Examples, and sufficient cross-linking did not occur, so that the desired deodorizing power could not be obtained.

Furthermore, even in Examples, it can be seen that the greater the number of carbon atoms in the alkyl group Ri, the better the deodorizing power.

**<Experimental Example 3> Antibacterial power measurement of antibacterial deodorant composition**

After 40 ml of artificial urine inoculated with 3,000 CFU/ml Proteus mirabilis was poured into 2 g of the antibacterial deodorant composition, the resulting mixture was cultured at 35°C for 12 hours. After the cultured solution was diluted with 160 ml of a physiological saline solution, samples serially diluted with the physiological saline solution were spread on an agar plate for calculation, and the results are shown in the following Table 5.

Specifically, 100 µm of the diluted sample was dropped on the agar plate and incubated at 30°C for about 24 hours, and then the number of bacteria was counted, and the antibacterial power was calculated and evaluated by the following equation from the number of bacteria and the number of bacteria of the control not surface-treated with the first compound (antibacterial deodorant monomer).

$$\text{Antibacterial power (\%)} = \{1 - (N_{\text{sample}})/(N_{\text{reference}})\} \times 100$$

($N_{sample}$: number of bacteria of sample containing antibacterial deodorant monomer, $N_{reference}$: number of bacteria of control containing no antibacterial deodorant monomer)

[Table 5]

| Antibacterial deodorant composition | Antibacterial power (%) |
|---|---|
| Example 1 | 99.0 |
| Example 2 | 97.5 |
| Example 3 | 99.2 |
| Example 4 | 90.5 |
| Example 5 | 92.1 |
| Example 6 | 91.1 |
| Comparative Example 1 | 25.0 |
| Comparative Example 2 | 80.0 |
| Comparative Example 3 | 88.8 |
| Comparative Example 4 | 89.0 |

(continued)

| Antibacterial deodorant composition | Antibacterial power (%) |
|---|---|
| Comparative Example 5 | 81.2 |
| Comparative Example 6 | 79.8 |
| Comparative Example 7 | 73.5 |
| Comparative Example 8 | 90.5 |

**<Experimental Example 4> Antibacterial power measurement of antibacterial deodorant composition**

After 50 ml of artificial urine inoculated with 3,000 CFU/ml E. coli was poured into 2 g of the antibacterial deodorant composition, the resulting mixture was cultured at 35°C for 12 hours. After the cultured solution was diluted with 150 ml of a physiological saline solution, samples serially diluted with the physiological saline solution were spread on an agar plate for calculation, and the results are shown in the following Table 6.

Specifically, 100 μm of the diluted sample was dropped on the agar plate and incubated at 30°C for about 24 hours, and then the number of bacteria was counted, and the antibacterial power was calculated and evaluated by the following equation from the number of bacteria and the number of bacteria of the control not surface-treated with the first compound (antibacterial deodorant monomer).

$$\text{Antibacterial power (\%)}=\{1-(N_{sample})/(N_{reference})\} \times 100$$

($N_{sample}$: number of bacteria of sample containing antibacterial deodorant monomer, $N_{reference}$: number of bacteria of control containing no antibacterial deodorant monomer)

[Table 6]

| Antibacterial deodorant composition | Antibacterial power (%) |
|---|---|
| Example 7 | >99.9 |
| Example 8 | >99.9 |
| Example 9 | >99.9 |
| Example 10 | >99.9 |
| Example 11 | >99.9 |
| Example 12 | >99.9 |
| Example 13 | >99.9 |
| Comparative Example 9 | 26.3 |
| Comparative Example 10 | 0 |

Through Tables 5 and 6, it can be known that a compound with a small number of carbon atoms in the alkyl group $R_1$ (Comparative Example 1), a compound (Comparative Example 2) different from the present invention, or a compound comprising no hydroxyl group (Comparative Example 3 or 4) was used, or a compound (Comparative Example 5) comprising a long alcohol group having 7 or more carbon atoms or a compound (Comparative Example 6) comprising only a long alkyl group having 10 or more carbon atoms was used, the antibacterial power was reduced, and in Comparative Example 7, the surface cross-linking reaction time was also shorter than that of Examples, and sufficient cross-linking did not occur, so that the desired deodorizing power could not be obtained.

In addition, even among the examples, it can be seen that a compound having two hydroxyl groups has a higher antibacterial power than a compound having one hydroxyl group, and the antibacterial power is further improved as the number of carbon atoms in the alkyl group $R_1$ increases.

Furthermore, it can be confirmed that when the content of the first compound of Chemical Formula 1 is less than 0.4 parts by weight based on 100 parts by weight of the entire second compound, the antibacterial power is not sufficiently exhibited.

**<Experimental Example 5> Ammonia deodorizing power measurement of antibacterial deodorant composition**

After 40 ml of artificial urine inoculated with 3,000 CFU/ml Proteus mirabilis was poured into 2 g of the antibacterial deodorant composition, the resulting mixture was cultured at 35°C for 12 hours. The ammonia content (ppm) of the cultured solution was measured using a 3M ammonia detector tube, and the results are shown in the following Table 7.

[Table 7]

| Antibacterial deodorant composition | Ammonia (ppm) |
|---|---|
| Example 1 | <10 |
| Example 2 | 50 |
| Example 3 | <10 |
| Example 4 | 160 |
| Example 5 | 150 |
| Example 6 | 150 |
| Comparative Example 1 | 500 |
| Comparative Example 2 | 380 |
| Comparative Example 3 | 200 |
| Comparative Example 4 | 200 |
| Comparative Example 5 | 350 |
| Comparative Example 6 | 400 |
| Comparative Example 7 | 450 |
| Comparative Example 8 | 200 |

Observing the results in Table 7, it can be seen that the ammonia content of Examples 1 to 6 comprising the antibacterial deodorant composition of the present invention was 160 ppm or less, which is much lower than those of Comparative Examples 1 to 8 in which 200 ppm to 500 ppm of ammonia was measured. Therefore, it can be confirmed that the antibacterial deodorant composition of the present invention has better deodorizing effect against ammonia than the compositions used in Comparative Examples 1-8. In particular, in the case of Examples 1 to 3 having two hydroxyl groups, the concentration of ammonia was measured to be 50 ppm or less, which was remarkably low.

**<Experimental Example 6> Determination of centrifuge retention capacity**

The centrifuge retention capacity (CRC) was determined in accordance with EDANA WSP 241.3. First, 1.5 g ($W_0$) of the antibacterial deodorant composition prepared above was uniformly placed in a non-woven fabric bag, the bag was sealed, and then the sealed bag was immersed in a physiological saline solution at room temperature. After 30 minutes had passed, moisture was removed from the bag for 3 minutes under a condition of 250 G using a centrifuge, and the mass $W_2$ (g) of the bag was measured. Alternatively, the mass $W_1$ (g) was measured after performing the experiment in the same manner as described above without using the antibacterial deodorant composition. Using each obtained mass, the centrifuge retention capacity (CRC) (g/g) was calculated according to the following equation.

[Equation 1]

$$CRC(g/g) = \{[W_2\,(g) - W_1\,(g)]/W_0\,(g)\} - 1$$

**<Experimental Example 7> Determination of absorbing under pressure**

For the absorbing under pressure (AUP), an absorbing under pressure of 0.7 psi was measured in accordance with EDANA WSP 242.3. First, a 400-mesh stainless steel wire mesh was mounted to the bottom of a plastic cylinder having an inner diameter of 60 mm. 1 g of ($W_0$) of the antibacterial deodorant composition prepared above was uniformly

sprayed on a wire mesh under conditions of room temperature and humidity of 50%, and a piston capable of further applying a load of 0.7 psi uniformly thereon was slightly smaller than an outer diameter of 60 mm and had no gaps with the inner wall of the cylinder, and the vertical movement thereof was not hindered. In this case, the weight $W_3$ (g) of the device was measured. A glass filter having a diameter of 90 mm and a thickness of 5 mm was placed inside a petri dish having a diameter of 150 mm and a physiological saline solution composed of 0.9 wt% sodium chloride was brought flush with the top surface of the glass filter. A sheet of filter paper with a diameter of 90 mm was placed thereon. The measuring device was placed on the filter paper and allowed a liquid to be absorbed under load for 1 hour. After 1 hour, the measuring device was lifted and its weight $W_4$ (g) was measured. Using each obtained mass, the absorbing under pressure (AUP)(g/g) was calculated according to the following equation.

[Equation 2]

$$AUP\ (g/g) = [W_4\ (g) - W_3\ (g)]/W_0\ (g)$$

The centrifuge retention capacity and absorbing under pressure measured in Experimental Examples 6 and 7 are shown in the following Table 8.

[Table 8]

| Antibacterial deodorant composition | Centrifuge retention capacity | Absorbing under pressure |
|---|---|---|
| Example 1 | 39 | 27 |
| Example 2 | 40 | 24 |
| Example 3 | 37 | 27 |
| Example 4 | 41 | 22 |
| Example 5 | 40 | 21 |
| Example 6 | 42 | 21 |
| Example 7 | 41 | 22 |
| Comparative Example 1 | 39 | 21 |
| Comparative Example 2 | 39 | 20 |
| Comparative Example 3 | 38 | 20 |
| Comparative Example 4 | 35 | 23 |
| Comparative Example 5 | 33 | 23 |
| Comparative Example 6 | 34 | 21 |
| Comparative Example 7 | 45 | 17 |
| Comparative Example 8 | 45 | 15 |

In summary, it can be seen that the antibacterial deodorant composition of the present invention not only provides excellent centrifugal retention capacity and absorbing under pressure, but also has the effect of improving antibacterial power and deodorizing power.

## Claims

1. An antibacterial deodorant composition comprising:

   a first compound of the following Chemical Formula 1; and
   a second compound different from the first compound,
   wherein the first compound is cross-linked to at least a portion of the second compound,
   a content of guaiacol is 300 ng or less, a content of 3-methylbutanal is 250 ng or less, and a content of diacetyl is 30 ng or less when the antibacterial deodorant composition is evaluated for deodorization, and

the deodorization evaluation is determined by the following Method A:

[Chemical Formula 1]

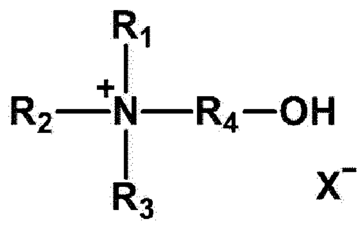

in Chemical Formula 1,

$R_1$ to $R_3$ are each independently an alkyl group having 1 to 12 carbon atoms, which is unsubstituted or substituted with a hydroxyl group,
at least one of $R_1$ to $R_3$ is an alkyl group having 8 to 12 carbon atoms,
$R_4$ is an alkylene group having 1 to 6 carbon atoms, and
X is a halogen,
[Method A]
After 1 g of the antibacterial deodorant composition is put into a 500 ml Lab bottle, 25 ml of artificial urine inoculated with microorganisms was injected into the composition, the resulting mixture was cultured at 35°C for 24 hours, and then guaiacol, 3-methylbutanal, and diacetyl components are each captured in an adsorption tube, and the mass of each captured component is analyzed using GC/MS.

2. The antibacterial deodorant composition of claim 1, wherein the antibacterial deodorant composition comprises the first compound in an amount of 0.4 parts by weight or more and 2.5 parts by weight or less based on 100 parts by weight of the entire second compound.

3. The antibacterial deodorant composition of claim 1, wherein at least one of $R_1$ and $R_2$ of Chemical Formula 1 is an alkyl group having 1 to 5 carbon atoms.

4. The antibacterial deodorant composition of claim 1, wherein the second compound is a super absorbent resin.

5. The antibacterial deodorant composition of claim 1, wherein the second compound comprises a hydrous gel polymer.

6. The antibacterial deodorant composition of claim 1, wherein the antibacterial deodorant composition is present in the form of particles having a sea-island structure.

7. The antibacterial deodorant composition of claim 1, wherein when the antibacterial deodorant composition is evaluated for antibacterial power, the antibacterial power is 90% or more, and the antibacterial power evaluation is determined by the following Method B:

[Method B]
After 40 ml of artificial urine inoculated with 3,000 CFU/ml bacteria is poured into 2 g of the antibacterial deodorant composition, the resulting mixture is cultured at 35°C for 12 hours, and after the cultured solution is diluted with 160 ml of a saline solution, samples serially diluted with the physiological saline solution are spread on an agar plate for calculation.

8. The antibacterial deodorant composition of claim 1, wherein the antibacterial deodorant composition has a centrifuge retention capacity of 30 g/g or more and 60 g/g or less.

9. The antibacterial deodorant composition of claim 1, wherein the antibacterial deodorant composition has an absorbing under pressure of 10 g/g or more and 40 g/g or less.

10. A method for preparing an antibacterial deodorant composition of any one of claims 1 to 9, the method comprising:

(a) preparing a mixture of the first compound of Chemical Formula 1 and a second compound different from the first compound; and
(b) cross-linking the mixture.

**11.** The method of claim 10, wherein Step (b) is performed at 150°C to 220°C for a period of time of more than 20 minutes.

**12.** The method of claim 10, wherein Step (b) is performed at 170°C to 200°C for a period of time of 30 minutes to 80 minutes.

**13.** A deodorant composition comprising a first compound of the following Chemical Formula 1:

[Chemical Formula 1]

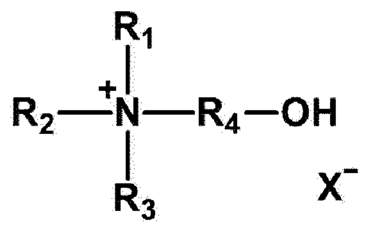

wherein, in Chemical Formula 1,

$R_1$ to $R_3$ are each independently an alkyl group having 1 to 12 carbon atoms, which is unsubstituted or substituted with a hydroxyl group,
at least one of $R_1$ to $R_3$ is an alkyl group having 8 to 12 carbon atoms,
$R_4$ is an alkylene group having 1 to 6 carbon atoms, and
X is a halogen.

**14.** The deodorant composition of claim 13, wherein the deodorant composition has a bacterial inhibition rate of 80% or more against at least one strain of Gram-positive bacteria and Gram-negative bacteria as evaluated by the following Method E:

[Method E]
25 ml of a broth type medium (Nutrient broth, BD DIFCP., 8 g/L) inoculated with 3,000 CFU/ml bacteria is transferred to a 50-ml conical tube, 0.01 g of the deodorant composition is added thereto, and then mixing is performed (vortexing). The well-mixed solution is cultured in a constant-temperature shaking water bath maintained at 35°C for 16 hours. After the cultured solution is diluted to 1/5 using a 1X phosphate buffered saline (PBS) buffer solution, absorbance (λ = 600 nm) is measured using a UV/Vis spectrophotometer. The measured absorbance is compared to that of the control, and the bacterial inhibition rate is calculated by the following equation. In this case, the control means a medium solution which does not contain the deodorant composition.

$$\text{Bacterial inhibition rate (\%)} = \{1-(A_{sample})/(A_{reference})\} \times 100$$

($A_{sample}$: absorbance of medium solution containing deodorant composition, $A_{reference}$: absorbance of medium solution containing no deodorant composition)

**15.** The deodorant composition of claim 14, wherein the strain of *E. coli* is determined by Method E, and the bacterial inhibition rate is 88% or more.

**16.** The deodorant composition of claim 14, wherein the strain of *Proteus mirabilis* is determined by Method E, and the bacterial inhibition rate is 83% or more.

[Figure 1]

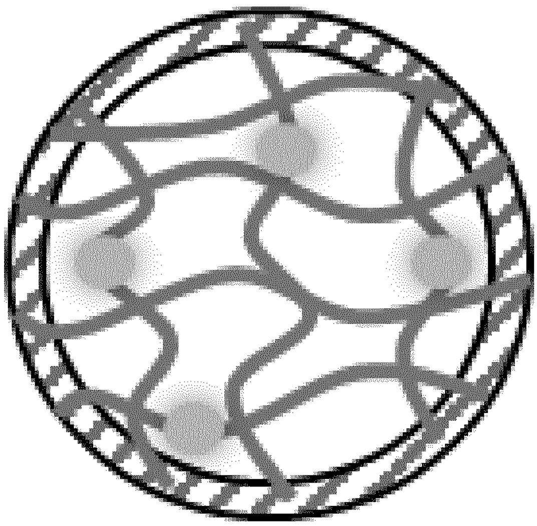

[Figure 2]

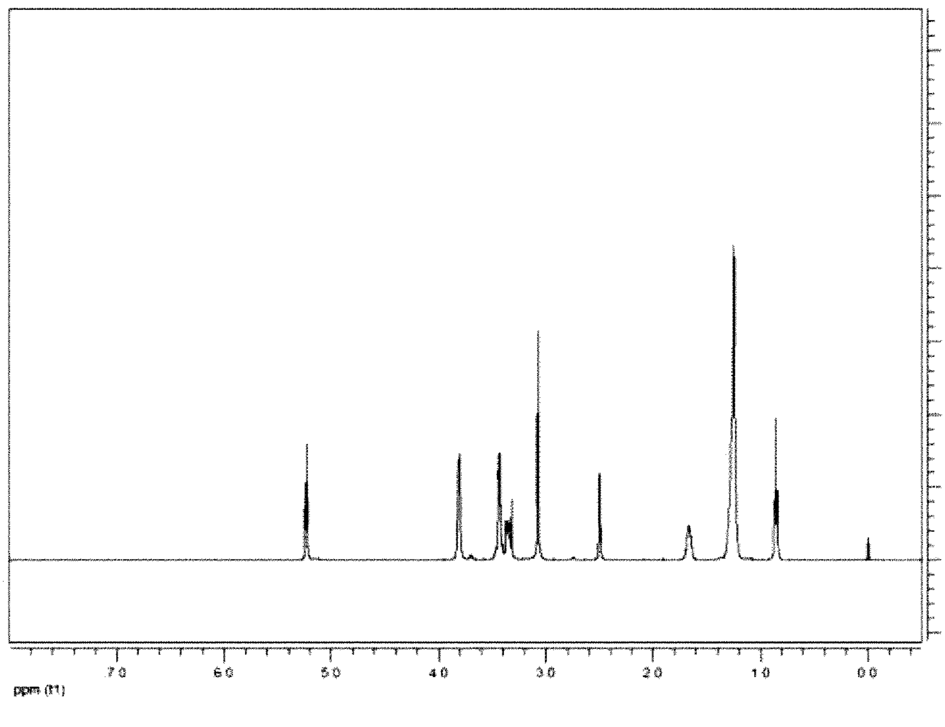

[Figure 3]

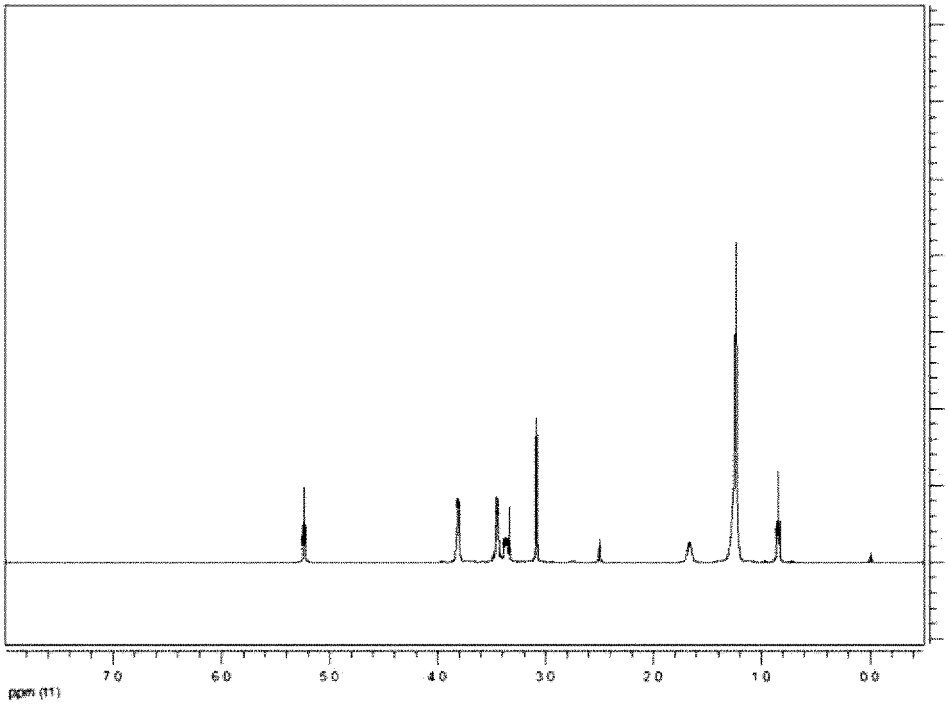


[Figure 4]

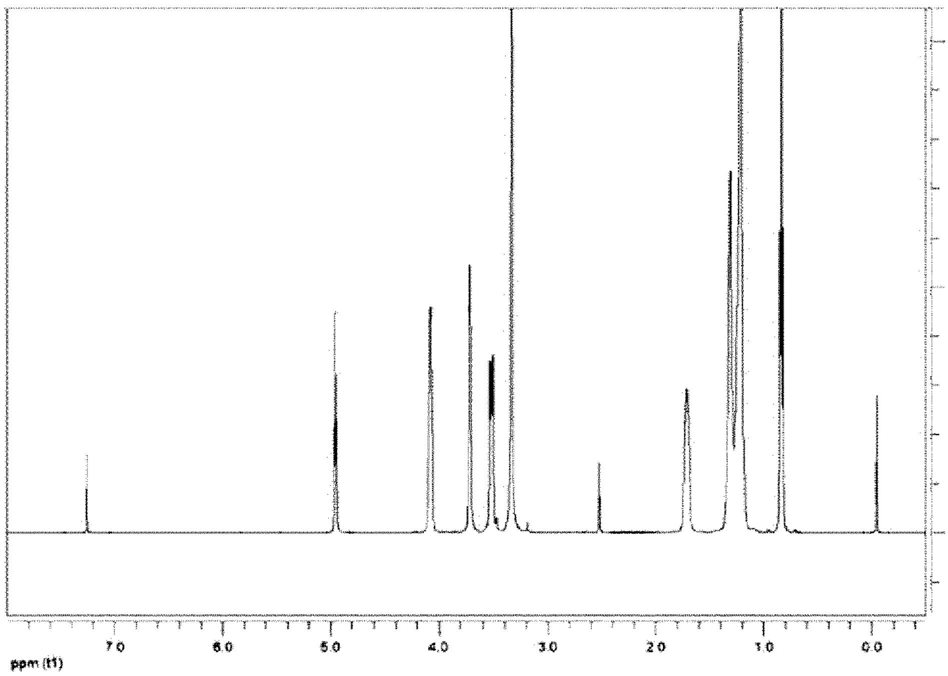

[Figure 5]

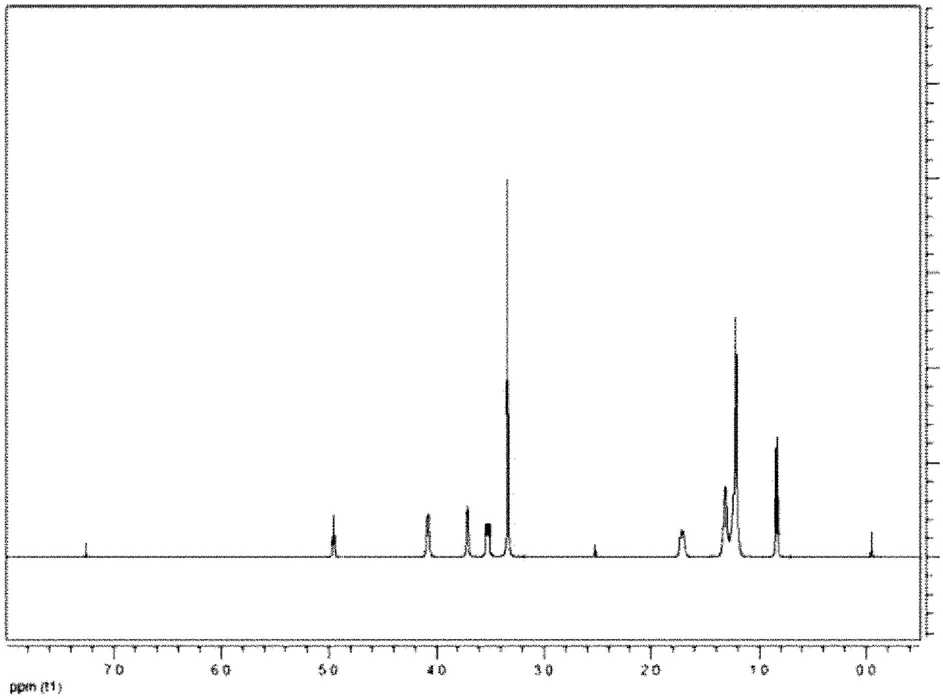


[Figure 6]

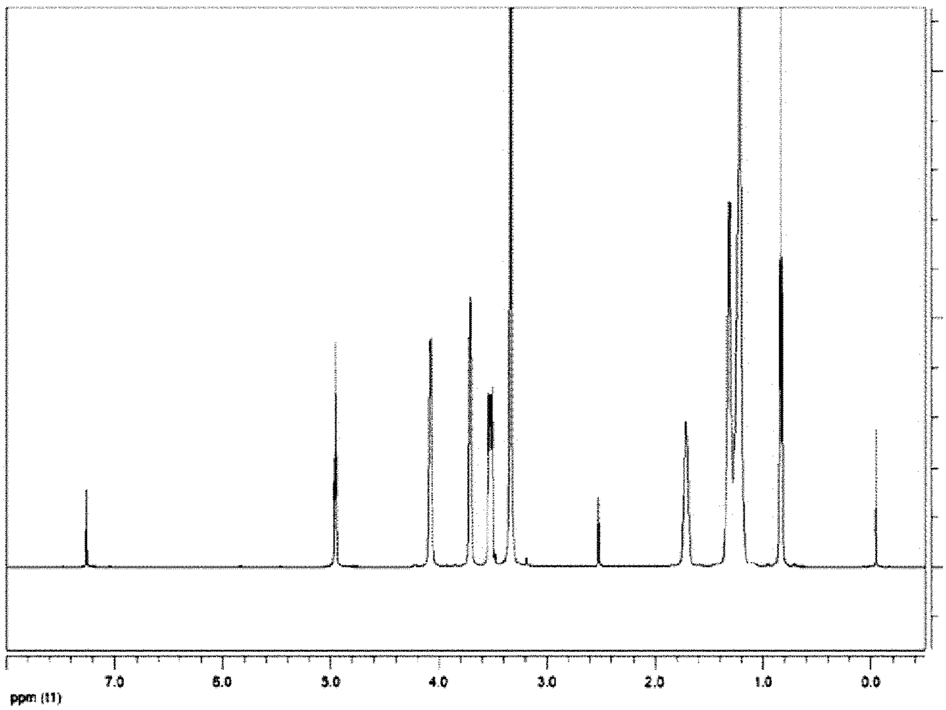

[Figure 7]

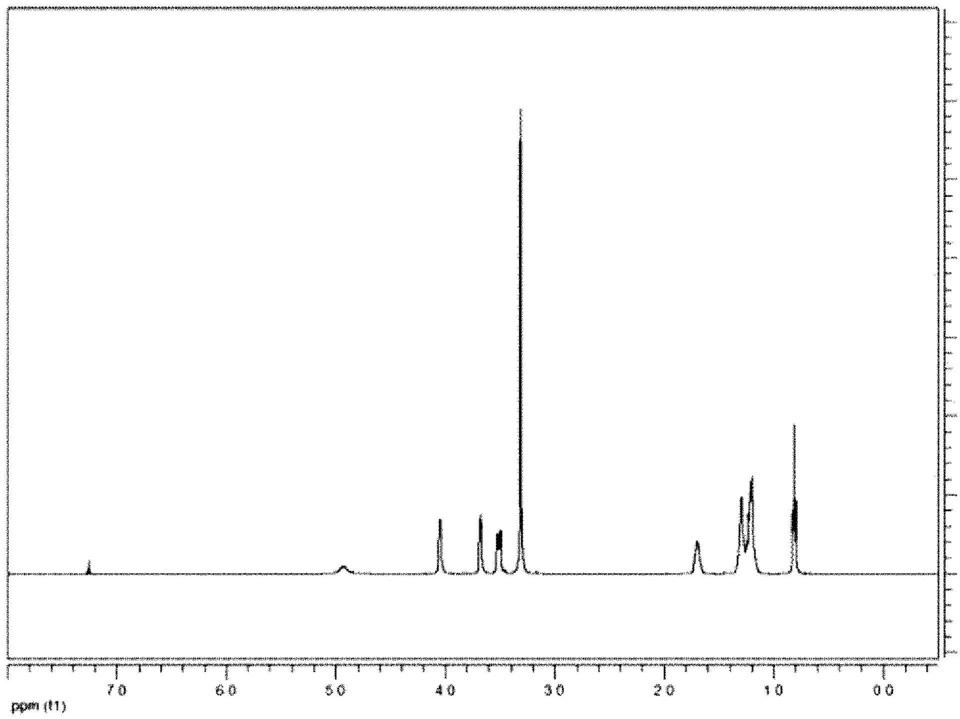


[Figure 8]

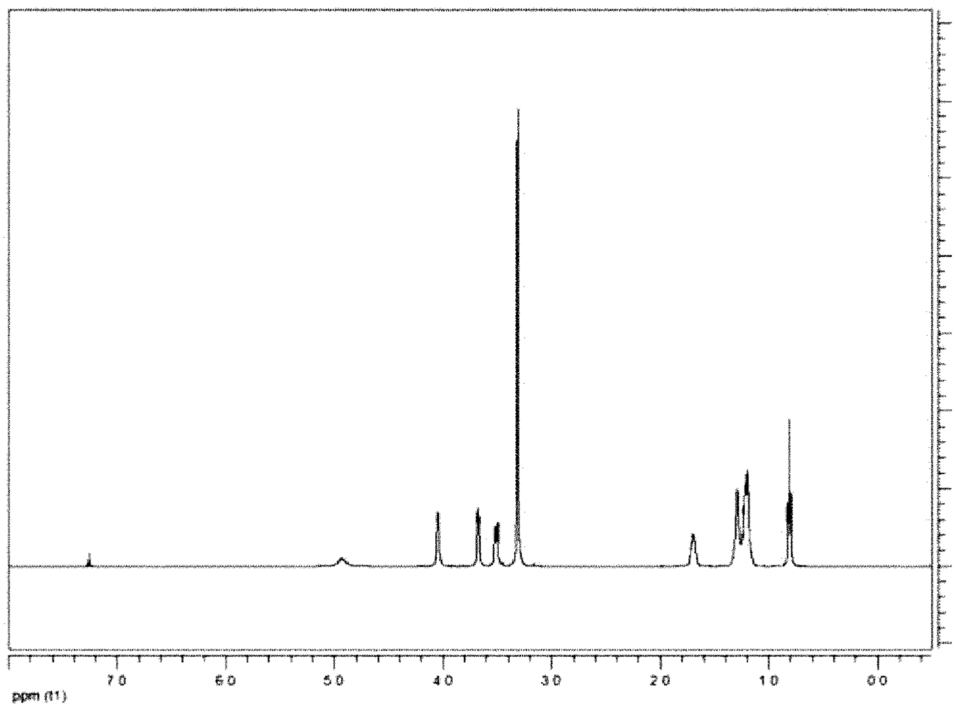

## INTERNATIONAL SEARCH REPORT

International application No.
**PCT/KR2022/009359**

**A. CLASSIFICATION OF SUBJECT MATTER**

**A61L 9/01**(2006.01)i; **A61L 9/012**(2006.01)i; **C08J 3/24**(2006.01)i

According to International Patent Classification (IPC) or to both national classification and IPC

**B. FIELDS SEARCHED**

Minimum documentation searched (classification system followed by classification symbols)

A61L 9/01(2006.01); A01N 25/30(2006.01); A01N 33/12(2006.01); C08F 220/06(2006.01); C08F 222/38(2006.01); C08J 3/12(2006.01); C08K 13/06(2006.01); C08K 5/20(2006.01); C08L 75/08(2006.01); C11D 1/86(2006.01); C11D 3/386(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus) & keywords: 고흡수성 수지(super absorbent polymer), 4급 암모늄(quaternary ammonium), 항균(antimicrobial), 소취(deodorization), 가교(crosslinking)

**C. DOCUMENTS CONSIDERED TO BE RELEVANT**

| Category* | Citation of document, with indication, where appropriate, of the relevant passages | Relevant to claim No. |
|---|---|---|
| Y<br>A | KR 10-2020-0073751 A (LG CHEM, LTD.) 24 June 2020 (2020-06-24)<br>See claims 1 and 4; paragraphs [0001], [0034], [0058], [0127] and [0226]; and table 1. | 1-12<br>13-16 |
| X<br>Y | CN 103992426 A (ZHEJIANG SATELLITE PETRO CHEMICAL CO., LTD.) 20 August 2014 (2014-08-20)<br>See claims 1 and 4; and paragraphs [0005] and [0036]. | 13-16<br>1-12 |
| A | US 2018-0084777 A1 (LONZA INC.) 29 March 2018 (2018-03-29)<br>See claims 1-22. | 1-16 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 October 2022** | **05 October 2022** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

INTERNATIONAL SEARCH REPORT

International application No.
PCT/KR2022/009359

C. DOCUMENTS CONSIDERED TO BE RELEVANT

| Category* | Citation of document, with indication, where appropriate, of the relevant passages | Relevant to claim No. |
|---|---|---|
| A | CN 105154249 A (CHINA RESEARCH INSTITUTE OF DAILY CHEMICAL INDUSTRY) 16 December 2015 (2015-12-16)<br>See entire document. | 1-16 |
| A | CN 104262946 B (DONGGUAN XIONGLIN NEW MATERIAL TECHNOLOGY CO., LTD.) 07 December 2016 (2016-12-07)<br>See entire document. | 1-16 |

Form PCT/ISA/210 (second sheet) (July 2019)

**INTERNATIONAL SEARCH REPORT**
**Information on patent family members**

International application No.
**PCT/KR2022/009359**

| Patent document cited in search report | | | Publication date (day/month/year) | Patent family member(s) | | | Publication date (day/month/year) |
|---|---|---|---|---|---|---|---|
| KR | 10-2020-0073751 | A | 24 June 2020 | None | | | |
| CN | 103992426 | A | 20 August 2014 | CN | 103992426 | B | 29 March 2017 |
| US | 2018-0084777 | A1 | 29 March 2018 | BR | 112019005791 | A2 | 18 June 2019 |
| | | | | CN | 109843054 | A | 04 June 2019 |
| | | | | EP | 3503726 | A1 | 03 July 2019 |
| | | | | WO | 2018-064021 | A1 | 05 April 2018 |
| CN | 105154249 | A | 16 December 2015 | CN | 105154249 | B | 31 August 2018 |
| CN | 104262946 | B | 07 December 2016 | CN | 104262946 | A | 07 January 2015 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020210110455


### Non-patent literature cited in the description


- *J Wound Ostomy Continence Nurs.,* 2019, vol. 46 (6), 519-523
- **REINHOLD SCHWALM.** UV Coatings: Basics, Recent Developments and New Application. Elsevier, 2007, 115
- **ODIAN.** Principle of Polymerization. Wiley, 1981, 203